# EUROPÄISCHE PATENTSCHRIFT

(11) **EP 3 292 133 B1**
(45) Veröffentlichungstag und Bekanntmachung des Hinweises auf die Patenterteilung: **26.06.2019**
(21) Anmeldenummer: 16722584.6
(22) Anmeldetag: 02.05.2016
(51) Int. Cl.: A61K 47/64, C07K 5/062, C07D 417/12, A61P 13/12, A61P 9/10, A61P 9/04

(54) **VERFAHREN ZUR HERSTELLUNG VON 2-{4-[2-({[2-(4-CHLORPHENYL)-1,3-THIAZOL-4-YL]METHYL}SULFANYL)-3,5-DICYAN-6-(PYRROLIDIN-1-YL)PYRIDIN-4-YL]PHENOXY}ETHYL-L-ALANYL-L-ALANINAT-MONOHYDROCHLORID**
METHOD FOR THE PREPARATION OF 2-{4-[2-({[2-(4-CHLOROPHENYL) -1,3-THIAZOL-4-YL]METHYL}SULFANYL)-3,5-DICYAN-6- (PYRROLIDIN-1-YL)PYRIDIN-4-YL]PHENOXY}ETHYL-L-ALANYL-L-ALANINATE HYDROCHLORIDE
PROCEDE DE FABRICATION DE 2-{4-[2-({[2-(4-CHLOROPHENYL)-1,3-THIAZOL-4-YL]METHYL}SULFANYL)-3,5-DICYAN-6-(PYRROLIDIN-1-YL)PYRIDIN-4-YL]PHENOXY}ETHYL-L-ALANYL-L-ALANINAT-HYDROCHLORIDE

(30) Priorität: 06.05.2015 EP 15166606; 29.10.2015 EP 15192030
(43) Veröffentlichungstag der Anmeldung: 14.03.2018
(73) Patentinhaber: Bayer Pharma Aktiengesellschaft, 13353 Berlin (DE)
(72) Erfinder: MAIS, Franz-Josef, 40591 Düsseldorf (DE); HEILMANN, Werner, 42327 Wuppertal (DE); OLENIK, Britta, 46242 Bottrop (DE); KEIL, Birgit, 40231 Düsseldorf (DE); BECKER, Guido, 47800 Krefeld (DE); MEIBOM, Daniel, 42113 Wuppertal (DE); KUHLMANN, Thomas, 50678 Köln (DE)
(74) Vertreter: BIP Patents
(86) Internationale Anmeldenummer: PCT/EP2016/059779
(87) Internationale Veröffentlichungsnummer: WO 2016/188711

(56) Entgegenhaltungen:
- WO-A1-2010/086101

## Beschreibung

Die vorliegende Anmeldung betrifft ein neues und verbessertes Verfahren zur Herstellung der Verbindung 2-{4-[2-({[2-(4-Chlorphenyl)-1,3-thiazol-4-yl]methyl}sulfanyl)-3,5-dicyan-6-(pyrrolidin-1-yl)pyridin-4-yl]-phenoxy}ethyl-L-alanyl-L-alaninat-Monohydrochlorid der Formel (I), 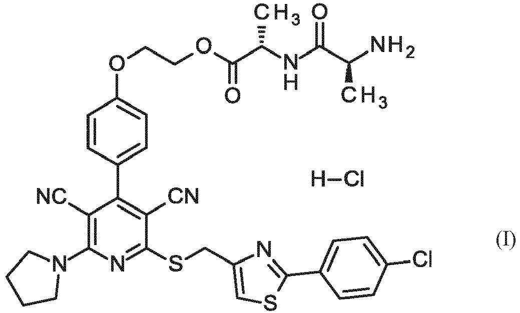 neue Vorstufen zu deren Herstellung, sowie die Herstellung und Verwendung der kristallinen Modifikation I von 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-alanyl-L-alaninat-Monohydrochlorid der Formel (I).

Die Verbindung der Formel (I) wirkt als partieller Adenosin A1 Rezeptor Agonist und kann als Mittel zur Prophylaxe und/oder Behandlung von kardiovaskulären Erkrankungen wie beispielsweise chronischer Herzinsuffizienz (worsening chronic heart failure), Angina pectoris und ischämischer Verletzung während eines akuten Koronarsyndroms eingesetzt werden.

2-{4-[2-({[2-(4-Chlorphenyl)-1,3-thiazol-4-yl]methyl}sulfanyl)-3,5-dicyan-6-(pyrrolidin-1-yl)pyridin-4-yl]-phenoxy}ethyl-L-alanyl-L-alaninat-Hydrochlorid und deren Herstellung sind in der WO 2010/086101 beschrieben (siehe dort Beispiel 44). Nachteilig an dem im Beispiel 44 durchgeführten Verfahren ist, dass ein Feststoff erhalten wird, dessen HCl-Gehalt stöchiometrisch nicht eindeutig definiert ist. Das bei der Nachstellung von Beispiel 44 erhaltene Material (siehe Beispiel 10 unten) ist amorph mit geringen kristallinen Anteilen. Der HCl-Gehalt in diesem Feststoff betrug ca. 1,7 mol HCl pro Mol des heterocyclischen Grundkörpers. Für eine Verwendung als Wirkstoff ist ein amorphes Material mit nicht exakt definierter stöchiometrischer Zusammensetzung nicht geeignet.

Es bestand daher die Aufgabe ein Verfahren zur Herstellung eines exakt definierten Monohydrochlorids der Formel (I) in reproduzierbarer kristalliner Form zu finden.

Weiterhin nachteilig an der dort beschriebenen Forschungssynthese ist die Tatsache, dass sich diese Synthese nicht in allen Schritten für die großtechnische Durchführung der Verfahren eignet, da viele Schritte in sehr hoher Verdünnung, mit sehr hohen Reagenz-Überschüssen und damit zu einer relativ geringen Gesamtausbeute verlaufen. Des Weiteren sind viele chromatographische Zwischenreinigungen notwendig, die in der Regel technisch sehr aufwendig sind und einen hohen Verbrauch an Lösungsmitteln erfordern, kostenintensiv sind und damit nach Möglichkeit vermieden werden sollten. Einige Stufen lassen sich aufgrund von sicherheits- und verfahrenstechnischen Vorgaben nicht realisieren, wie z.B. die Verwendung von 1-Hydroxybenzotriazol (HOBt) als Reagenz oder Diethylether als Lösungsmittel.

Es bestand daher auch der Bedarf nach einer großtechnisch praktikablen Synthese, die die Verbindung der Formel (I) reproduzierbar in hoher Gesamtausbeute, niedrigen Gestehungskosten, hoher Reinheit und einer pharmazeutisch brauchbaren Kristallform liefert und allen regulatorischen Anforderungen entspricht, um die klinischen Prüfungen mit Wirkstoff zu beliefern und für eine spätere behördliche Einreichung verwendet zu werden.

Mit der vorliegenden Erfindung wurde ein Verfahren gefunden, das Material der Formel (I) in exakt definierter stöchiometrischer Form als kristallines Produkt liefert. Weiterhin wurde eine sehr effiziente Synthese gefunden, die es erlaubt die oben genannten Anforderungen zu erfüllen.

In WO 2010/086101 ist die Forschungssynthese der Verbindung 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-alanyl-L-alaninat-Hydrochlorid offenbart. Ausgehend von 2-Amino-6-({[2-(4-chlorphenyl)-1,3-thiazol-4-yl]methyl}sulfanyl)-4-[4-(2-hydroxyethoxy)phenyl]pyridin-3,5-dicarbonitril (II), auch bekannt unter dem INN Capadenoson, wird die Zielverbindung nach dem Verfahren im Stand der Technik über 6 Stufen mit einer Ausbeute von 43 % der Theorie hergestellt. Die Zielverbindung wird in WO 2010/086101 zwar als Feststoff erhalten, ein definiertes Kristallisations-Verfahren der Endstufe zur Einstellung der exakten Monohydrochlorid Stöchiometrie und zur Herstellung einer pharmazeutisch brauchbaren Kristallform bzw. zur Polymorphie-Einstellung ist bisher nicht beschrieben.

Das nachfolgende Schema 1 zeigt das bekannte Verfahren zur Herstellung der Verbindung der Formel (I).

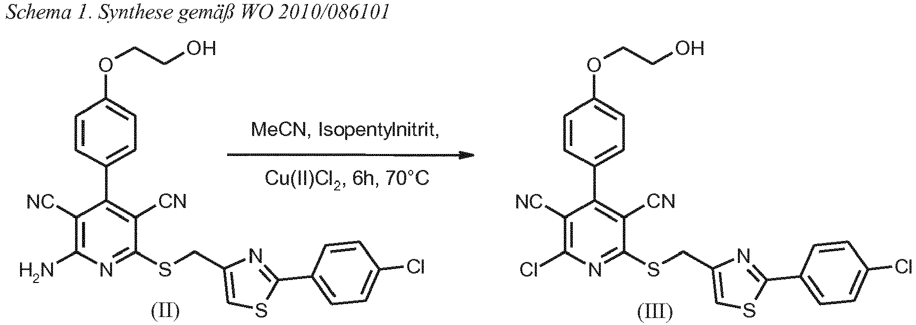

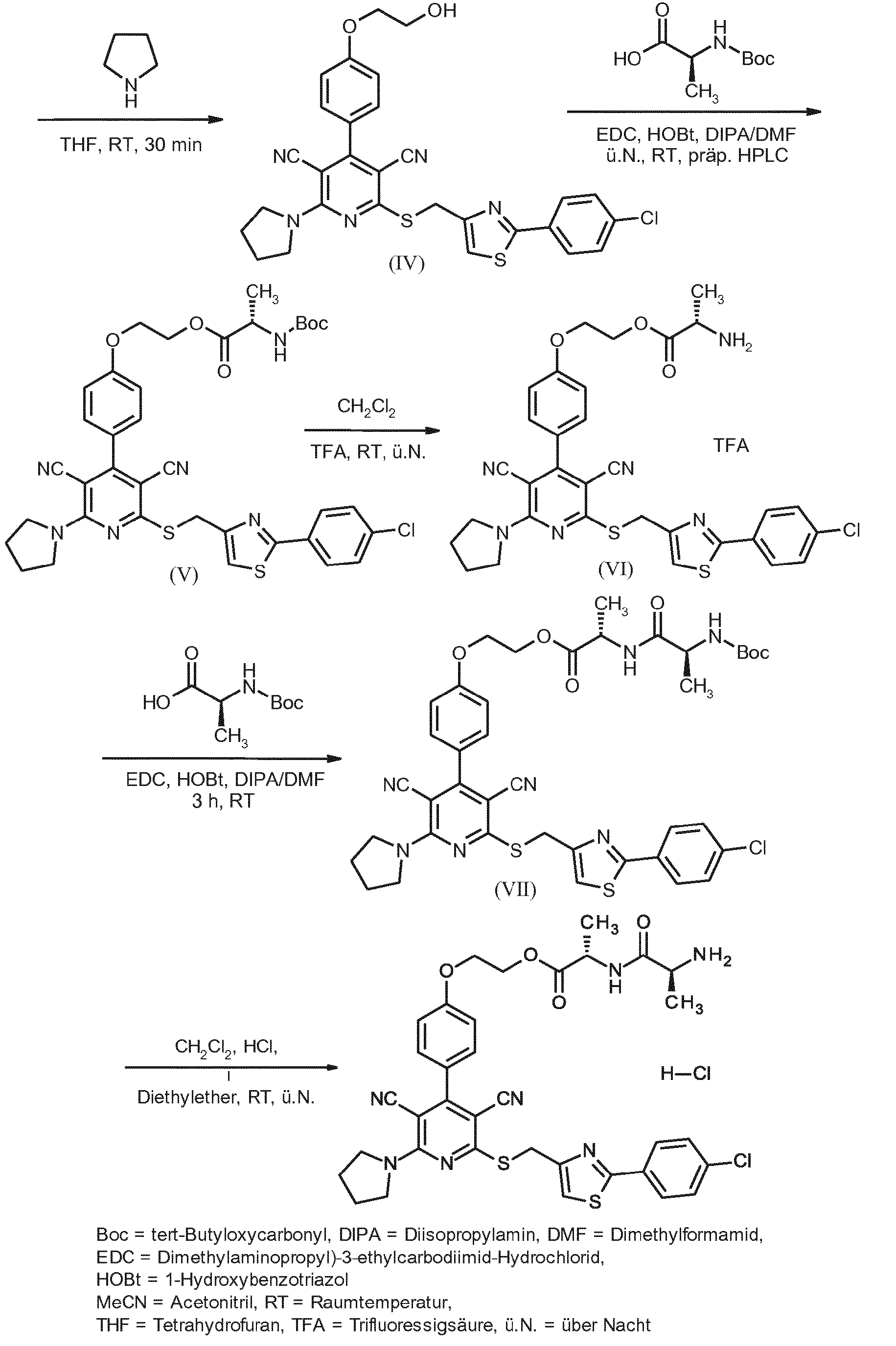

Das Verfahren läuft über 6 Stufen, wovon die Stufe zum Produkt (V) chromatographisch aufgereinigt wird. Für die Kupplung der Aminosäurebausteine zum Aufbau der Seitenkette wird 1-Hydroxybenzotriazol (HOBt) als Reagenz eingesetzt, das großtechnisch aus Sicherheitsgründen (Explosionsgefahr) nicht eingesetzt werden kann. Die Verwendung von Diethylether als Lösungsmittel ist aufgrund des niedrigen Flammpunktes ebenfalls großtechnisch nicht ohne aufwendige zusätzliche Sicherheitseinrichtungen möglich. Weiterhin besonders nachteilig ist, dass die Zielverbindung nach der in WO 2010/086101 beschriebenen Methode (Beispiel 44) nicht in einer pharmazeutisch brauchbaren definierten Kristallform, sondern als amorphes Material mit nicht exakt definierter Zusammensetzung bezüglich des HCl-Gehaltes anfällt.

Schema 2 veranschaulicht das neue erfindungsgemäße Verfahren, welches die Verbindung der Formel (I) in exakt definierter stöchiometrischer Form als kristallines Produkt in 5 Stufen in 55,3 % der Theorie Gesamtausbeute ohne die Notwendigkeit einer chromatographischen Aufreinigung liefert.

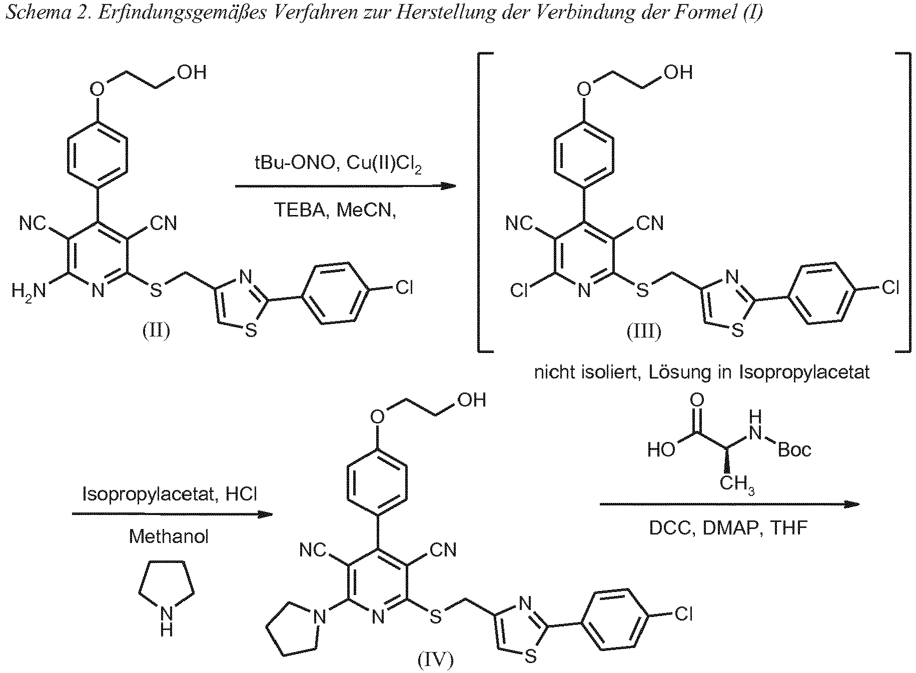

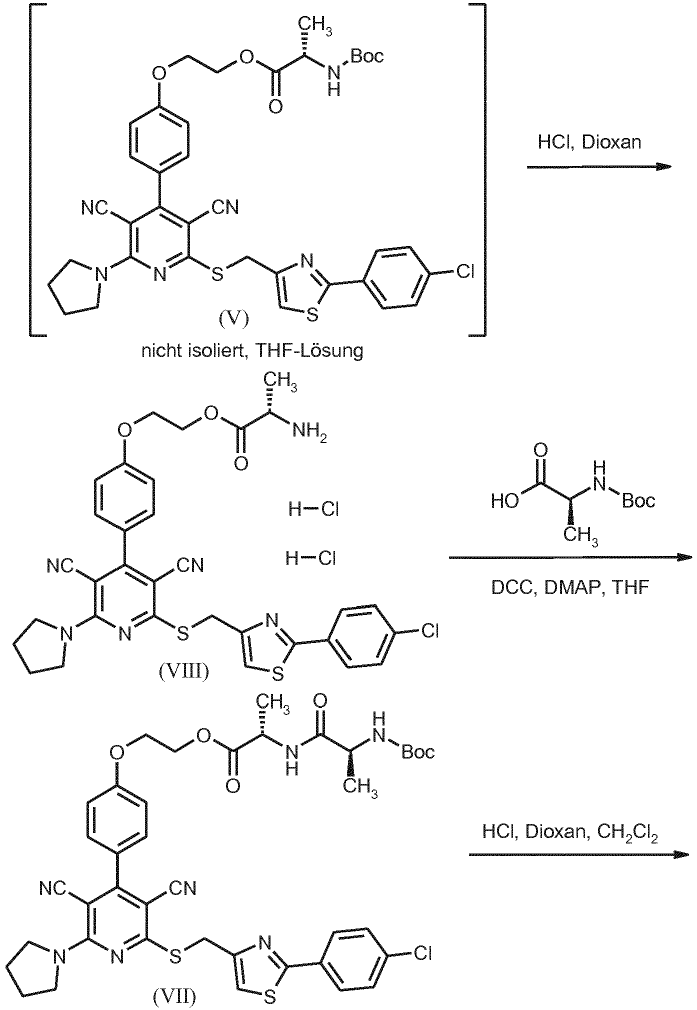

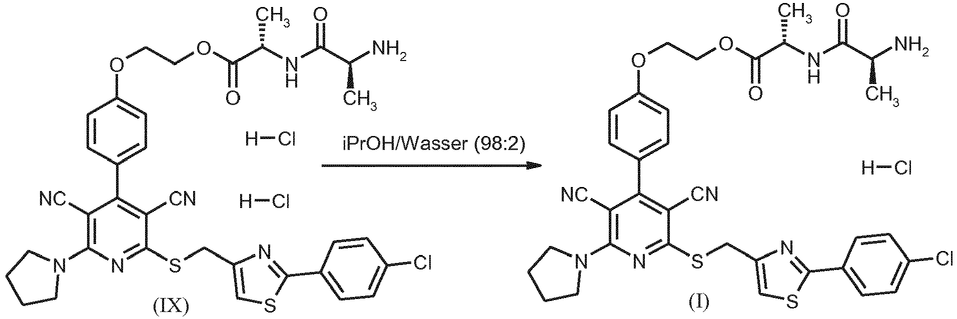

Das Chlormercaptothiazolopyridin (III) und das geschützte Boc-Alaninat (V) werden nicht isoliert, sondern direkt in Lösung weiter umgesetzt. In einer Variante kann das Verfahren so geführt werden, dass das auch geschützte Boc-Dialaninat (VII) ebenfalls nicht isoliert, sondern direkt in Lösung weiter umgesetzt wird. In diesem Fall besteht der Gesamtprozess nur noch aus 4 isolierten Stufen (mit einer Gesamtausbeute von ca. 75 %) anstelle von 6 Stufen im Stand der Technik (mit einer Gesamtausbeute von ca. 43 %). In Schema 3 ist das erfindungsgemäße Verfahren unter Berücksichtigung der isolierten Stufen dargestellt.

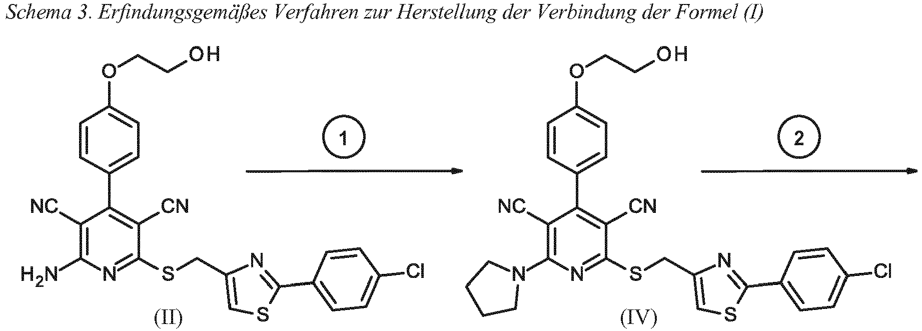

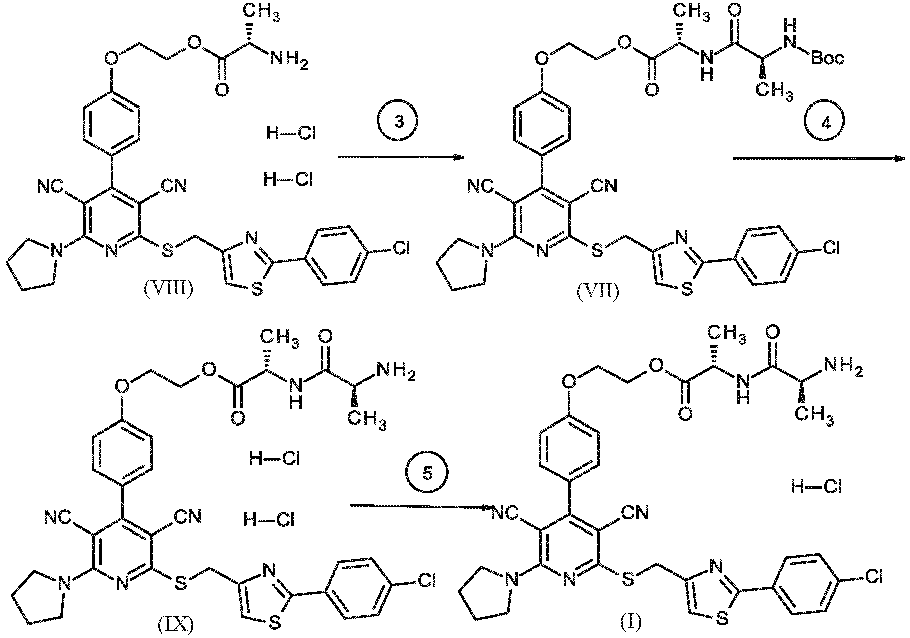

Ein wichtiger Vorteil des erfindungsgemäßen Verfahrens ist die Bereitstellung der Verbindung der Formel (I) in exakt definierter stöchiometrischer Form als kristallines Produkt, das für die Verwendung als pharmazeutischer Wirkstoff geeignet ist.

Im Folgenden werden die einzelnen Stufen des erfindungsgemäßen Verfahrens zur Herstellung der Verbindung der Formel (I) gemäß Schema 3 diskutiert. Alternativen, die durch die Isolierung der Verbindung der Formel (III) bzw. durch die Nichtisolierung der Verbindung der Formel (VII) gekennzeichnet sind, werden ebenfalls diskutiert.

Die Ausgangssubstanz der Formel (II) ist in WO 03/053441 beschrieben. Diese Verbindung der Formel (II) wird erhalten, indem man 2-[4-(2-Hydroxyethoxy)benzyliden]malononitril (XI) mit Cyanthioacetamid (XII) und 4-(Chlormethyl)-2-(4-chlorphenyl)-1,3-thiazol (XIV) umsetzt. Das substituierte Malononitril (XI) wird dabei durch Umsetzung von 4-(2-Hydroxyethoxy)benzaldehyd (X) mit Malononitril erhalten. Das Phenylthiazol (XIV) wird durch Umsetzung von 4-Chlorthiobenzamid (XIII) mit 1,3-Dichloraceton erhalten. Diese Synthese ist in Schema 4 zusammengefasst.

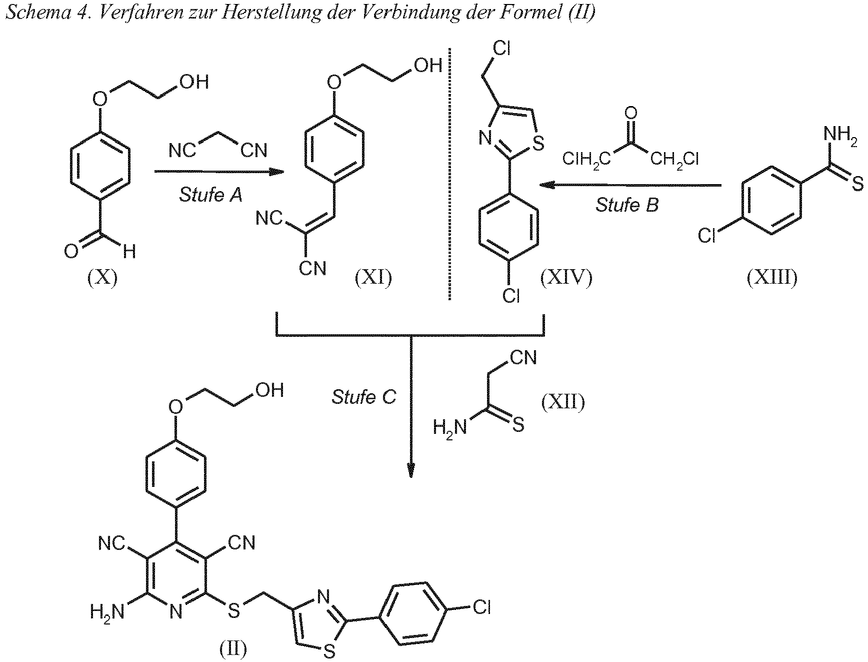

Bei diesem Verfahren ist insbesondere nachteilig, dass die Umsetzung von dem substituierten Malononitril (XI) mit dem Chlormethylchlorphenylthiazol (XIV) und dem Cyanthioacetamid (XII) (Stufe C) als Eintopfreaktion unter Zugabe aller drei Reaktionspartner direkt nacheinander durchgeführt wird. Dies führt in der Technik zu relativ niedrigen Ausbeuten (68%) mit relativ schlechter Produktqualität (ca. 95% Gehalt).

Erfindungsgemäß wird in der Stufe C bei der Herstellung der Verbindung der Formel (II) statt Tributylamin/Methanol nun Triethylamin/Methanol als Base/Lösungsmittel-Gemisch verwendet. Zudem wird der Prozess als vorteilhafte Eintopfreaktion in einer zweistufigen, dem chemischen Mechanismus angepassten Fahrweise durchgeführt. Zunächst lässt man die Reaktionspartner substituiertes Malononitril (XI) mit Cyanthioacetamid (XII) in Gegenwart von Triethylamin zu einer Zwischenstufe reagieren. Danach wird Chlormethylchlorphenylthiazol (XIV) zugegeben und bei leicht erhöhter Temperatur (40-50 °C) das Zielmolekül (II) gebildet. Hierdurch kann eine um 5% höhere Ausbeute (siehe Beispiel 1) erzielt werden.

Die Nummerierung der Stufen 1 bis 5 bezieht sich im Folgenden auf die Darstellung in Schema 3.

### Stufe 1

Stufe 1 des erfindungsgemäßen Verfahrens wurde dahin gehend optimiert, dass das Chlormercaptothiazolopyridin (III) als Zwischenstufe nicht isoliert, sondern in Lösung direkt weiter umgesetzt wird.

Zunächst wird 2-Amino-6-({[2-(4-chlorphenyl)-1,3-thiazol-4-yl]methyl}sulfanyl)-4-[4-(2-hydroxyethoxy)-phenyl]pyridin-3,5-dicarbonitril (II) in einem Lösungsmittel wie Acetonitril (alternativ verwendbar: Proprionitril, Butyronitril, Isobutyronitril; bevorzugt wird Acetonitril verwendet) in Anwesenheit eines Phasentransferkatalysators wie Benzyltriethylammoniumchlorid (alternativ verwendbar: Tetrabutylammoniumchlorid, Trimethylbenzylammoniumchlorid, Tributylbenzylammoniumchlorid; bevorzugt wird Benzyltriethylammoniumchlorid verwendet), in Anwesenheit eines Oxidationsmittels wie tert-Butylnitrit (alternativ verwendbar: Isoamylnitrit bzw. Isopentylnitrit, Butylnitrit, Isobutylnitrit; besonders bevorzugt werden tert-Butylnitrit, Isoamylnitrit bzw. Isopentylnitrit verwendet) und in Anwesenheit von Kupfer(II)chlorid umgesetzt. Das Zwischenprodukt Chlormercaptothiazolopyridin (III) wird nach Rühren z.B. in Isopropylacetat und verdünnter Salzsäure in der organischen Phase erhalten.

Im weiteren Verlauf der Synthese wird z.B. die Isopropylacetat-Lösung der Verbindung der Formel (III) mit Methanol (alternativ verwendbar: kurzkettige aliphatische Alkohole wie Methanol, Ethanol, Propanol, Isopropanol; bevorzugt wird Methanol verwendet) verdünnt und mit Pyrrolidin umgesetzt. Die Verbindung der Formel (IV) wird kristallin erhalten.

Im ersten Teilschritt werden typischerweise pro Mol an Verbindung der Formel (II) je 1,5 bis 4 mol, bevorzugt 1,8 bis 3 mol, besonders bevorzugt 2 Mol an Phasentransferkatalysator, je 1,5 bis 4 mol, bevorzugt 1,8 bis 3 mol, besonders bevorzugt 2 Mol an dem Nitrit und je 2 bis 5 mol, bevorzugt 2,5 bis 4 mol, besonders bevorzugt 3 Mol Kupfer(II)chlorid eingesetzt.

Die Ausgangstoffe Phasentransferkatalysator, Lösungsmittel und Verbindung der Formel (II) werden zunächst bei Raumtemperatur zusammengegeben. Nach Zugabe von Kupfer(II)chlorid wird auf 40 °C bis zum Siedepunkt des Lösungsmittel, bevorzugt 40 bis 60 °C, besonders bevorzugt 50 °C erwärmt und nach Zugabe des Nitrits (z.B. tert-Butylnitrit) für 2 bis 12 h, bevorzugt 3 bis 6 h besonders bevorzugt 4 h bei 50 °C weiter gerührt. Am Ende der Reaktion wird wieder auf Raumtemperatur (20 bis 30 °C) abgekühlt.

Die Aufarbeitung erfolgt durch Verrühren mit Isopropylacetat (alternativ verwendbar: Ethylacetat, Propylacetat, Butylacetat, Ethylpropionat; bevorzugt wird Isopropylacetat verwendet) und verdünnter Salzsäure (5 bis 20 % in Wasser, bevorzugt 5 bis 10 % in Wasser, besonders bevorzugt 7 % in Wasser) und anschließende Klärfiltration.

Im zweiten Teilschritt werden typischerweise pro Mol an Chlormercaptothiazolopyridin (III) je 3 bis 10 Mol, bevorzugt 3,5 bis 7 Mol, besonders bevorzugt 4 Mol an Pyrrolidin eingesetzt.

Die Zugabe von Pyrrolidin erfolgt durch leichte Gegenkühlung, so dass die Temperatur von 35 °C, bevorzugt 30 °C, besonders bevorzugt 25 °C nicht übersteigt. Anschließend wird zunächst 1 bis 5 h, bevorzugt 1,5 bis 3 h, besonders bevorzugt 2 h bei dieser Temperatur und dann über Nacht (10 bis 24 h, bevorzugt 15 bis 20 h) bei 50 °C bis zum Rückfluss des Lösungsmittels, bevorzugt bei 60 bis 70 °C, bzw. besonders bevorzugt in Methanol am Rückfluss (ca. 66 °C) gerührt.

Die Aufarbeitung erfolgt durch Abkühlung auf 0 bis 10 °C, bevorzugt ca. 5 °C. Das Produkt der Formel (IV) wird filtriert, gewaschen und getrocknet.

Die Ausbeute beträgt durch diese Reaktionsführung ca. 84 % über beide Teilschritte, während die in der Literatur bekannte Synthese nur ca. 56 % Ausbeute über beide Stufen liefert.

### Alternative mit Isolierung des Chlormercaptothiazolopyridin (III)

Alternativ zu dieser Eintopfreaktion kann die Reaktion auch mit Isolierung des Chlormercaptothiazolopyridin der Formel (III) erfolgen. Durch die erfindungsgemäße Reaktionsführung wird in beiden Teilreaktionen die Ausbeute gesteigert, von ca. 69 % auf ca. 79 % im ersten Teilschritt und von ca. 81 % auf ca. 91 % im zweiten Teilschritt.

Wie beim Eintopfverfahren wird zunächst 2-Amino-6-({[2-(4-chlorphenyl)-1,3-thiazol-4-yl]methyl}sulfanyl)-4-[4-(2-hydroxyethoxy)phenyl]pyridin-3,5-dicarbonitril (II) in einem Lösungsmittel wie Acetonitril (alternativ verwendbar: Proprionitril, Butyronitril, Isobutyronitril; bevorzugt wird Acetonitril verwendet) in Anwesenheit eines Phasentransferkatalysators wie Benzyltriethylammoniumchlorid (alternativ verwendbar: Tetrabutylammoniumchlorid, Trimethylbenzylammoniumchlorid, Tributylbenzylammoniumchlorid; bevorzugt wird Benzyltriethylammoniumchlorid verwendet), in Anwesenheit eines Oxidationsmittels wie tert-Butylnitrit (alternativ verwendbar: Isoamylnitrit bzw. Isopentylnitrit, Butylnitrit, Isobutylnitrit; besonders bevorzugt wird tert-Butylnitrit, Isoamylnitrit bzw. Isopentylnitrit verwendet) und in Anwesenheit von Kupfer(II)chlorid umgesetzt. Das Zwischenprodukt Chlormercaptothiazolopyridin (III) wird nach Rühren in Essigsäureethylester und Wasser in der organischen Phase erhalten. Nach Destillation und Umkristallisation aus Methanol wurde die Verbindung der Formel (III) erhalten.

Bei dieser Verfahrensführung werden pro Mol an Verbindung der Formel (II) 1,5 bis 4 mol, bevorzugt 1,8 bis 3 mol, besonders bevorzugt 2 Mol an Phasentransferkatalysator, je 1,5 bis 4 mol, bevorzugt 1,8 bis 3 mol, besonders bevorzugt 2 Mol an dem Nitrit und je 2 bis 5 mol, bevorzugt 2,5 bis 4 mol, besonders bevorzugt ca. 3 Mol Kupfer(II) chlorid eingesetzt.

Die Ausgangstoffe Phasentransferkatalysator, Lösungsmittel und Verbindung der Formel (II) werden zunächst bei Raumtemperatur zusammengegeben. Nach Zugabe von Kupfer(II)chlorid wird auf 40 °C bis zum Siedepunkt des Lösungsmittel, bevorzug 40 bis 60 °C, besonders bevorzugt 50 °C erwärmt und nach Zugabe des Nitrits (z.B. tert-Butylnitrit) für 2 bis 12 h, bevorzugt 3 bis 6 h, besonders bevorzugt 4 h bei 50 °C weiter gerührt. Am Ende der Reaktion wird wieder auf Raumtemperatur (20 bis 30 °C) abgekühlt.

Die Aufarbeitung erfolgt durch Verrühren mit Essigsäureethylester (alternativ verwendbar: Isopropylacetat, Propylacetat, Butylacetat, Ethylpropionat; bevorzugt wird Ethylacetat oder Isopropylacetat verwendet, besonders bevorzugt Ethylacetat) und Wasser. Die organischen Phasen werden anschließend destilliert. Aus dem Rückstand wird die Verbindung der Formel (III) nach Umkristallisation aus Methanol (alternativ verwendbar: kurzkettige aliphatische Alkohole wie Methanol, Ethanol, Propanol, Isopropanol; bevorzugt wird Methanol verwendet) erhalten.

Im weiteren Verlauf der Synthese wird die isolierte Verbindung der Formel (III) in Methanol (alternativ verwendbar: kurzkettige aliphatische Alkohole wie Methanol, Ethanol, Propanol, Isopropanol; bevorzugt wird Methanol verwendet) suspendiert und mit Pyrrolidin umgesetzt. Die Verbindung der Formel (IV) wird kristallin erhalten.

Typischerweise wird pro Mol an isoliertem Chlormercaptothiazolopyridin (III) je 3 bis 10 Mol, bevorzugt 3,5 bis 7,5 Mol an Pyrrolidin eingesetzt.

Die Zugabe von Pyrrolidin erfolgt durch leichte Gegenkühlung, so dass die Temperatur 35 °C, bevorzugt 30 °C besonders bevorzugt 25 °C nicht übersteigt. Anschließend wird zunächst 1 bis 5 h, bevorzugt 1,5 bis 3 h, besonders bevorzugt 2 h bei dieser Temperatur und dann über Nacht (10 bis 24 h, bevorzugt 15 bis 20 h) bei 50 °C bis zum Rückfluss des Lösungsmittels, bevorzugt bei 60 bis 70 °C, bzw. besonders bevorzugt in Methanol am Rückfluss (ca. 65 bis 66 °C) gerührt.

Die Aufarbeitung erfolgt durch Abkühlung auf 0 bis 10 °C, bevorzugt ca. 5 °C. Das Produkt der Formel (IV) wird filtriert, gewaschen und getrocknet.

### Stufe 2

Stufe 2 des erfindungsgemäßen Verfahrens wurde dahin gehend optimiert, dass das geschützte Boc-Alaninat (V) als Zwischenstufe nicht isoliert, sondern in Lösung direkt weiter umgesetzt wird. Bei der Durchführung dieser Stufe werden für die großtechnische Synthese besonders gut geeignete Bedingungen gewählt. Die Auswahl der Reaktionsparameter liefert bei dieser Amid-Kupplung mit anschließender Abspaltung der Schutzgruppe quantitative Ausbeuten an Alaninat-Dihydrochlorid der Formel (VIII).

Zunächst werden die Verbindung der Formel (IV) und Boc-L-Alanin in einem Lösungsmittel wie Tetrahydrofuran (alternative verwendbar: Dioxan, Methyltetrahydrofuran; bevorzugt wird Tetrahydrofuran verwendet) vorgelegt und dann in Anwesenheit von Dicyclohexylcarbodiimid (DCC) in Kombination mit 4-(Dimethylamino)-pyridin gerührt.

In diesem Teilschritt werden typischerweise pro Mol an Verbindung der Formel (IV) je 1 bis 3 mol, bevorzugt 1,2 bis 2 mol, besonders bevorzugt 1,3 bis 1,5 Mol an Boc-L-Alanin, 0,2 mol bis 1,5 mol, bevorzugt 0,4 bis 1 mol besonders bevorzugt ca. 0,5 Mol an 4-(Dimethylamino)-pyridin und 1 bis 3 mol, bevorzugt 1,2 bis 2 mol, besonders bevorzugt ca. 1,5 Mol an DCC eingesetzt.

Die Ausgangstoffe werden zunächst bei 10 bis 35 °C, bevorzugt 20 bis 30 °C, bevorzugt Raumtemperatur zusammengegeben und anschließend über Nacht (10 bis 24 h, bevorzugt 15 bis 20 h) bei dieser Temperatur gerührt.

Die Aufarbeitung erfolgt durch Absaugen fester Bestandteile und Nachwaschen mit dem verwendeten Lösungsmittel wie Tetrahydrofuran. Anschließend wird das Filtrat auf einen Teil der Gesamtmenge von 15 bis 30 %, bevorzugt ca. 20 % der Gesamtmenge eingeengt.

Im zweiten Teilschritt dieser Stufe 2 werden typischerweise pro Mol an nicht isoliertem geschütztem Boc-Alaninat (V) 10 bis 20 mol, bevorzugt 11 bis 15 mol, besonders bevorzugt ca. 12 Mol Salzsäure (in Form einer 4 M Lösung in Dioxan) eingesetzt.

Die Zugabe der Salzsäure erfolgt durch leichte Gegenkühlung, so dass die Temperatur von 15 bis 30 °C, bevorzugt 20-25 °C nicht übersteigt. Anschließend wird 5 bis 20 h, bevorzugt 10 bis 15 h, besonders bevorzugt ca. 12 h bei 15 bis 30 °C, bevorzugt 20-25 °C gerührt. Die Aufarbeitung erfolgt durch Filtration, Waschen und Trocknen des ausgefallenen Feststoffs.

Die Ausbeute ist quantitativ über beide Teilschritte. Die formal über zu 100 % berechnete Ausbeute ist durch Reste an Reagenzien und Lösungsmitteln sowie durch Dimethylaminopyridin Hydrochlorid, das im isolierten Produkt enthalten ist, bedingt. Diese Verunreinigungen stellen keine Qualitätsminderung der Zwischenstufe Alaninat-Dihydrochlorid (VIII) dar, weil sämtliche Reagenzien in der nächsten Stufe erneut eingesetzt werden und die Folgestufen Boc-Dialaninat (VII) bzw. Dialaninat-Dihydrochlorid (IX) komplett gereinigt werden.

### Stufe 3

Die Stufe 3 des erfindungsgemäßen Verfahrens wurde dahin gehend optimiert, dass eine Isolierung und Reinigung des geschützten Boc-Dialaninats der Formel (VII) ohne chromatographische Aufarbeitung erfolgt.

Dazu wird zunächst die Verbindung der Formel (VIII) und 4-(Dimethylamino)-pyridin (DMAP) in einem Lösungsmittel wie Tetrahydrofuran (THF) (alternative verwendbar: Dioxan, Methyltetrahydrofuran; bevorzugt wird Tetrahydrofuran verwendet) vorgelegt. Nach Zugabe von Boc-L-Alanin und Dicyclohexylcarbodiimid (DCC) wird die Reaktionsmischung gerührt.

Typischerweise werden pro Mol an Verbindung der Formel (VIII) je 1 bis 3 mol, bevorzugt 1,1 bis 2 mol, besonders bevorzugt 1,2 bis 1,5 Mol an Boc-L-Alanin, 1 bis 4 mol, bevorzugt 1,5 bis 3 mol, besonders bevorzugt 2 Mol an 4-(Dimethylamino)-pyridin und 1 bis 3 mol, bevorzugt 1,1 bis 2 mol, besonders bevorzugt ca. 1,2 Mol an DCC eingesetzt.

Die Ausgangstoffe Verbindung der Formel (VIII), DMAP und THF werden zunächst bei 10 bis 35 °C, bevorzugt 20 bis 30 °C, bevorzugt 20 bis 25 °C zusammengegeben, mit Boc-L-Alanin versetzt und nach Zugabe von DCC für 2 bis 12 h, bevorzugt 6 bis 8 h, besonders bevorzugt ca. 4 h bei dieser Temperatur gerührt.

Die Aufarbeitung erfolgt durch Absaugen fester Bestandteile und Nachwaschen mit 10 bis 20%-iger, bevorzugt ca. 15%-iger Lösung von Ammoniumchlorid in Wasser. Die organische Phase wird dann mehrfach mit Dioxan versetzt und abdestilliert. Das Boc-geschützte Dialaninat (VII) wird nach Kristallisation durch Zugabe von Diisopropylether in kristalliner Form erhalten.

### Stufe 4

Zur Durchführung der Stufe 4 wird zunächst das geschützte Boc-Dialaninat (VII) in Dichlormethan in der Hitze gelöst, filtriert und überschüssiges Dichlormethan wird abdestilliert. Der Sumpf wird mit Diisopropylether verdünnt und mit 10 bis 20 mol, bevorzugt 11 bis 15 mol besonders bevorzugt ca. 12 Mol Salzsäure je Mol der Verbindung (VII) (in Form einer 4 M Lösung in Dioxan) versetzt.

Die Zugabe der Salzsäure erfolgt so, dass die Innentemperatur von 15 bis 30 °C, bevorzugt 20 bis 25 °C nicht übersteigt. Anschließend wird 5 bis 24 h, bevorzugt 10 bis 20 h, besonders bevorzugt ca. 12 bis 16 h bei dieser Temperatur gerührt.

Die Aufarbeitung erfolgt durch Filtration des ausgefallenen Feststoffs.

Die Gesamtausbeute über die Stufe 3 und Stufe 4 ist sehr hoch (96,9 %), während die in der Literatur bekannte Synthese nur 88 % Ausbeute über 2 Stufen liefert. Dabei wird als Ausgangsstoff das Alaninat-Trifluoracetat der Formel (VI) anstelle des Alaninat-Dihydrochlorids (VIII) verwendet.

### Alternative zu Stufe 3 und Stufe 4 ohne Zwischenisolierung des Boc-Dialaninats (VII)

In einer alternativen Ausgestaltung wurde die Stufe 3 des erfindungsgemäßen Verfahrens dahin gehend optimiert, dass das geschützte Boc-Dialaninat (VII) als Zwischenstufe nicht isoliert, sondern in Lösung direkt weiter umgesetzt wird. Bei der Durchführung dieser Stufe werden für die großtechnische Synthese besonders gut geeignete Bedingungen gewählt. Die Auswahl der Reaktionsparameter liefert bei dieser Amid-Kupplung mit anschließender Abspaltung der Schutzgruppe fast quantitative Ausbeuten an Dialaninat-Dihydrochlorid der Formel (IX).

Zunächst werden die Verbindung der Formel (VIII) und 4-(Dimethylamino)-pyridin (DMAP) in einem Lösungsmittel wie Tetrahydrofuran (THF) (alternativ verwendbar: Dioxan, Methyltetrahydrofuran; bevorzugt wird Tetrahydrofuran verwendet) vorgelegt. Nach Zugabe von Boc-L-Alanin und Dicyclohexylcarbodiimid (DCC) wird die Reaktionsmischung gerührt.

Typischerweise werden pro Mol an Verbindung der Formel (VIII) je 1 bis 3 mol, bevorzugt 1,1 bis 2 mol, besonders bevorzugt 1,2 bis 1,5 Mol an Boc-L-Alanin, 1 bis 4 mol, bevorzugt 1,5 bis 3 mol, besonders bevorzugt 2 Mol an 4-(Dimethylamino)-pyridin und 1 bis 3 mol, bevorzugt 1,1 bis 2 mol, besonders bevorzugt ca. 1,2 Mol an DCC eingesetzt.

Die Ausgangstoffe Verbindung der Formel (VIII), DMAP und THF werden zunächst bei 10 bis 35 °C, bevorzugt 20 bis 30 °C, bevorzugt 20 bis 25 °C zusammengegeben, mit Boc-L-Alanin versetzt und nach Zugabe von DCC für 2 bis 12 h, bevorzugt 6 bis 8 h, besonders bevorzugt ca. 4 h bei dieser Temperatur gerührt.

Die Aufarbeitung erfolgt durch Absaugen fester Bestandteile und Nachwaschen mit 10 bis 20%-iger, bevorzugt ca. 15%-iger Lösung von Ammoniumchlorid in Wasser.. Die organische Phase wird dann mehrfach mit Dioxan versetzt und abdestilliert. Das geschützte Boc-Dialaninat (VII) wird dann in Dioxan-Lösung im nächsten Teilschritt eingesetzt.

Im zweiten Teilschritt dieser Stufe 3 werden typischerweise pro Mol an nicht isoliertem geschütztem Boc-Dialaninat (VII) 10 bis 20 mol, bevorzugt 11 bis 15 mol, besonders bevorzugt ca. 12 Mol Salzsäure [je Mol der Verbindung (VII)] (in Form einer 4 M Lösung in Dioxan) zugegeben .

Die Zugabe der Salzsäure erfolgt so, dass die Innentemperatur von 15 bis 30 °C, bevorzugt 20 bis 25 °C nicht übersteigt. Anschließend wird 5 bis 24 h, bevorzugt 10 bis 20 h, besonders bevorzugt ca. 12 bis 16 h bei dieser Temperatur gerührt.

Die Aufarbeitung erfolgt durch Filtration des ausgefallenen Feststoffs.

Die Ausbeute ist fast quantitativ (98 %) über beide Teilschritte, während die in der Literatur bekannte Synthese nur 88 % Ausbeute über 2 Stufen liefert.

### Stufe 5

Stufe 5 des erfindungsgemäßen Verfahrens ist eine gänzlich neue Stufe, in der das Hydrochlorid der Formel (I) aus dem Dialaninat-Dihydrochlorid (IX) unter ganz bestimmten Bedingungen erhalten wird. Dabei wird der stöchiometrische Gehalt an HCl exakt auf die Monohydrochlorid Stufe eingestellt und das Produkt der Formel (I) wird in der erfindungsgemäßen kristallinen Modifikation I erhalten.

In Stufe 5 wird das Dialaninat-Dihydrochlorid (IX) in einem 10 bis 25-fachen, bevorzugt 12 bis 20-fachen, besonders bevorzugt in einem ca. 15-fachen Überschuss eines Alkohol/Wasser-Gemisches (Wasseranteil 0,5 bis 5 vol-%, bevorzugt 1 bis 3 vol-%, besonders bevorzugt ca. 2 vol-%, Alkohol: Isopropanol oder n-Propanol, bevorzugt Isopropanol) gerührt, abfiltriert, mit dem Alkohol gewaschen und getrocknet. Alternativ kann auch nur der Alkohol ohne Wasserzusatz verwendet werden.

Das Dialaninat-Dihydrochlorid (IX) wird bei 10 bis 35 °C, bevorzugt 15 bis 30 °C, besonders bevorzugt bei 23 bis 28 °C mit dem Isopropanol-Wasser-Gemisch versetzt und dann 6 bis 96 h, bevorzugt 12 bis 84 h, besonders bevorzugt 18 bis 72 h bei dieser Temperatur gerührt.

Die Aufarbeitung erfolgt durch Filtration, mehrfachem Waschen mit dem Alkohol und Trocknen. Wenn in dem bevorzugten Lösungsmittel Isopropanol gerührt wird, wird durch Filtration und Wäsche zunächst ein Isopropanol-Solvat geringer thermischer Stabilität isoliert, das durch die nachfolgende Trocknung vollständig in die kristalline Form der Modifikation (I) übergeht.

Da die Verbindung der Formel (I) in Form einer Tablette entwickelt wird, besteht ein hoher Bedarf dafür, dass man die isolierte Verbindung der Formel (I) reproduzierbar in einer exakt definierten HCl-Stöchiometrie und in einer definierten kristallinen Form isoliert, so dass man reproduzierbare Wirkstoffgehalte bei der Tablettenproduktion und eine reproduzierbare Bioverfügbarkeit gewährleisten kann. Es wurde überaschenderweise gefunden, dass man die Verbindung der Formel (I) aus Isopropanol bzw. n-Propanol/Wasser (98:2) kristallisieren kann, wobei reproduzierbar die kristalline Modifikation I entsteht, welche einen Schmelzpunkt von 156 bis 166 °C besitzt.

Zur weiteren Reinigung kann das isolierte Feuchtprodukt vor der Trocknung nochmal mit dem Isopropanol bzw. n-Propanol/Wasser Gemisch (Wasseranteil 0,5 bis 5 vol-%, bevorzugt 1 bis 3 vol-%, besonders bevorzugt ca. 2 vol-%) in einem 10 bis 25-fachen, bevorzugt 12 bis 20-fachen, besonders bevorzugt in einem ca. 15-fachen Überschuss für 1 bis 36 h, bevorzugt 12 bis 24 h gerührt werden. Anschließend wird abfiltriert, gewaschen und im Vakuum getrocknet.

Ein Gegenstand der vorliegenden Erfindung ist die Verbindung der Formel (I) in kristalliner Form der Modifikation I, dadurch gekennzeichnet, dass das Röntgendiffraktogramm der Verbindung Peakmaxima des 2 Theta Winkels bei 6.5, 8.7 und 24.3 zeigt.

Ein Gegenstand der vorliegenden Erfindung ist die Verbindung der Formel (I) in kristalliner Form der Modifikation I, dadurch gekennzeichnet, dass das Röntgendiffraktogramm der Verbindung Peakmaxima des 2 Theta Winkels bei 6.5, 8.7, 18.3, 19.9, 20.7, 23.5 und 24.3 zeigt.

Ein weiterer Gegenstand der vorliegenden Erfindung ist die Verbindung der Formel (I) in kristalliner Form der Modifikation I, dadurch gekennzeichnet, dass das Raman-Spektrum der Verbindung Bandenmaxima bei 2907, 1004 und 598 cm⁻¹ zeigt.

Ein weiterer Gegenstand der vorliegenden Erfindung ist die Verbindung der Formel (I) in kristalliner Form der Modifikation I, dadurch gekennzeichnet, dass das Raman-Spektrum der Verbindung Bandenmaxima bei 2907, 1539, 1515, 1182, 1004 und 598 cm⁻¹ zeigt.

Ein weiterer Gegenstand der vorliegenden Erfindung ist die Verbindung der Formel (I) in kristalliner Form der Modifikation I, dadurch gekennzeichnet, dass das Raman-Spektrum der Verbindung Bandenmaxima bei 2907, 1539, 1515, 1394, 1245, 1182, 1004 und 598 cm⁻¹ zeigt.

Ein weiterer Gegenstand der vorliegenden Erfindung ist ein Verfahren zur Herstellung der Verbindung der Formel (I) in kristalliner Form der Modifikation I, dadurch gekennzeichnet, dass das Dichlorid der Formel (IX) in Isopropanol bzw. n-Propanol/Wasser (98:2 v/v) für mehrere Stunden gerührt, anschließend filtriert, gewaschen und im Vakuum getrocknet wird.

Bevorzugtes Lösungsmittel für das Verfahren zur Herstellung der Verbindung der Formel (I) in kristalliner Form der Modifikation I ist Isopropanol/Wasser (98:2 v/v).

Ein bevorzugter Temperaturbereich für das Verfahren zur Herstellung der Verbindung der Formel (I) in kristalliner Form der Modifikation I ist von 20 bis 30 °C.

Ein weiterer Gegenstand der vorliegenden Erfindung ist die Verbindung der Formel (I) in kristalliner Form der Modifikation I wie oben beschrieben zur Behandlung von Krankheiten.

Ein weiterer Gegenstand der vorliegenden Erfindung ist ein Arzneimittel enthaltend eine Verbindung der Formel (I) in kristalliner Form der Modifikation I wie oben beschrieben und keine größeren Anteile einer anderen Form der Verbindung der Formel (I) als die kristalline Form der Modifikation I wie oben beschrieben. Arzneimittel enthaltend eine Verbindung der Formel (I) in kristalliner Form der Modifikation I wie oben beschrieben in mehr als 90 Gewichtsprozente bezogen auf die Gesamtmenge der enthaltenen Verbindung der Formel (I) in kristalliner Form der Modifikation I wie oben beschrieben.

Ein weiterer Gegenstand der vorliegenden Erfindung ist die Verwendung der Verbindung der Formel (I) in kristalliner Form der Modifikation I wie oben beschrieben zur Herstellung eines Arzneimittels zur Behandlung von Herz-Kreislauferkrankungen.

Ein weiterer Gegenstand der vorliegenden Erfindung ist die Methode zur Behandlung von Herz-Kreislauferkrankungen durch Verabreichung einer wirksamen Menge einer Verbindung der Formel (I) in kristalliner Form der Modifikation I wie oben beschrieben.

Ein weiterer Gegenstand der vorliegenden Erfindung ist ein Verfahren zur Herstellung der Verbindung der Formel (I) in kristalliner Form der Modifikation I, dadurch gekennzeichnet, dass das Dialaninat-Dihydrochlorid (IX) in einem Überschuss an Isopropanol/Wasser-Gemisch (98:2 v/v) bei Raumtemperatur rührt und die Verbindung der Formel (I) in der kristallinen Modifikation I nach dem Trocknen erhalten wird.

Ein weiterer Gegenstand der vorliegenden Erfindung ist ein Verfahren zur Herstellung von Verbindung (I), dadurch gekennzeichnet, dass man
[A] in Stufe 1 in einer Eintopfreaktion die Verbindung der Formel (II) 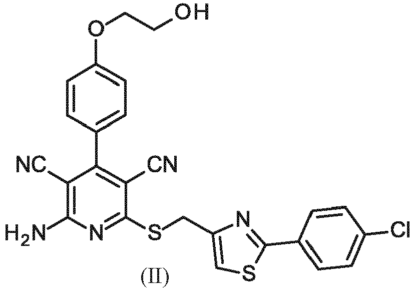 in Gegenwart eines Lösungsmittels, eines Phasentransferkatalysator, eines Nitrits und Kupfer(II)chlorids umsetzt und das erhaltene Zwischenprodukt der Formel (III) 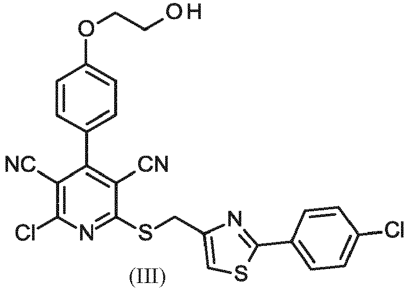 ohne Isolierung, d.h. in Lösung, mit Pyrrolidin zur Verbindung der Formel (IV) 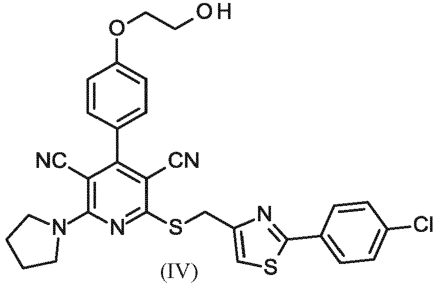 umsetzt, und
[B] diese so erhaltene Verbindung der Formel (IV) in einer Stufe 2 in einer Eintopfreaktion mit Boc-L-Alanin in Gegenwart des Kondensationsmittels Dicyclohexylcarbodiimid (DCC) in Kombination mit 4-(Dimethylamino)-pyridin und in Gegenwart eines Lösungsmittels zum geschützten Boc-Alaninat (V) 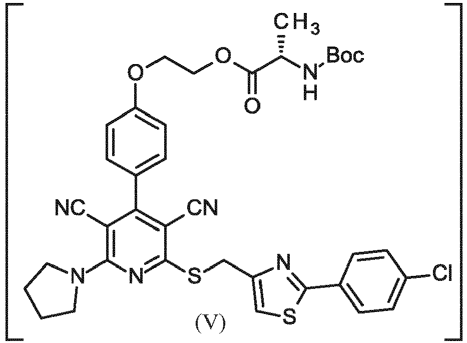 umsetzt, welches ohne Isolierung, d.h. in Lösung, mit Salzsäure zum Alaninat-Dihydrochlorid der Formel (VIII) 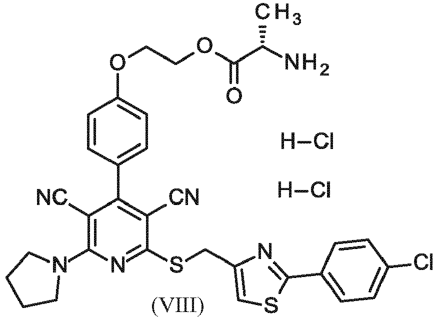 umgesetzt wird, und
[C] diese so erhaltene Verbindung der Formel (VIII) in einer Stufe 3 in Kombination mit 4-(Dimethylamino)-pyridin und in Gegenwart eines Lösungsmittels mit Boc-L-Alanin in Gegenwart des Kondensationsmittels Dicyclohexylcarbodiimid (DCC) zum geschützten Boc-Dialaninat (VII) 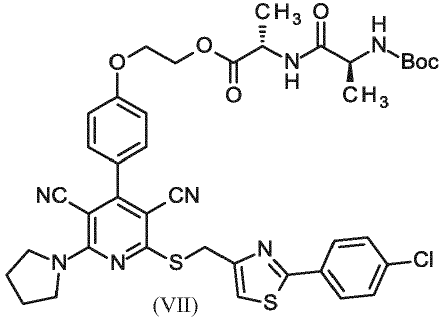 umsetzt, und
[D] diese so erhaltene Verbindung der Formel (VII) in einer Stufe 4 in Gegenwart eines Lösungsmittels mit Salzsäure zum Dialaninat-Dihydrochlorid der Formel (IX) 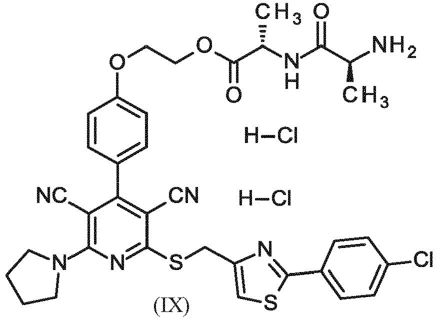 umgesetzt, und
[E] diese so erhaltene Verbindung der Formel (IX) in einer Stufe 5 in einem Überschuss an Isopropanol oder n-Propanol/Wasser-Gemisch (98:2) bei Raumtemperatur rührt und nach dem Trocknen die Verbindung der Formel (I) in der kristallinen Modifikation I erhält.

Ein weiterer Gegenstand der vorliegenden Erfindung ist ein Verfahren zur Herstellung von Verbindung (I) in der kristallinen Modifikation I, dadurch gekennzeichnet, dass man in Stufe 1 das Zwischenprodukt der Formel (III) isoliert, das heißt als Feststoff erhält, bevor es weiter umgesetzt wird.

Ein weiterer Gegenstand der vorliegenden Erfindung ist ein Verfahren zur Herstellung von Verbindung (I) in der kristallinen Modifikation I, dadurch gekennzeichnet, dass man in Stufe 2 das geschützte Boc-Alaninat der Formel (V) isoliert, das heißt als Feststoff erhält, bevor es weiter umgesetzt wird.

Ein weiterer Gegenstand der vorliegenden Erfindung ist ein Verfahren zur Herstellung von Verbindung (I) in der kristallinen Modifikation I, dadurch gekennzeichnet, dass man das in Stufe 3 erhaltene geschützte Boc-Dialaninat der Formel (VII) nicht isoliert, sondern direkt weiter umsetzt.

Ein weiterer Gegenstand der vorliegenden Erfindung ist ein Verfahren zur Herstellung von Verbindung (I), dadurch gekennzeichnet, dass in Stufe 1 Benzyltriethylammoniumchlorid als Phasentransferkatalysator eingesetzt wird.

Ein weiterer Gegenstand der vorliegenden Erfindung ist ein Verfahren zur Herstellung von Verbindung (I), dadurch gekennzeichnet, dass in Stufe 1 tert-Butylnitrit als Oxidationsmittel eingesetzt wird.

Ein weiterer Gegenstand der vorliegenden Erfindung ist ein Verfahren zur Herstellung von Verbindung (I), dadurch gekennzeichnet, dass in Stufe 1 Benzyltriethylammoniumchlorid als Phasentransferkatalysator und tert-Butylnitrit als Oxidationsmittel eingesetzt wird.

Ein weiterer Gegenstand der vorliegenden Erfindung ist des Dialaninat-Dihydrochlorid der Formel (IX)

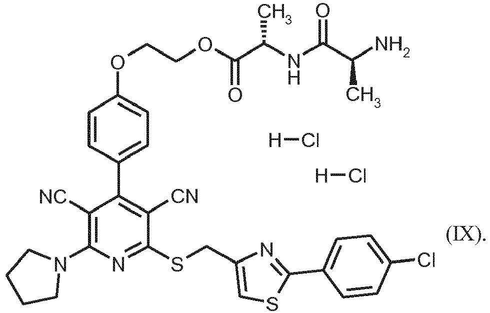

Ein weiterer Gegenstand der vorliegenden Erfindung ist ein Verfahren zur Herstellung des Dialaninat-Dihydrochlorids der Formel (IX), dadurch gekennzeichnet, dass man das Alaninat-Dihydrochlorid der Formel (VIII) mit Boc-L-Alanin in Gegenwart eines Kondensationsmittel (z.B. Dicyclohexylcarbodiimid) und in Gegenwart von 4-(Dimethylamino)-pyridin in einem Lösungsmittel (z.B. Tetrahydrofuran) umsetzt und das so erhaltene geschützte Boc-Dialaninat (VII) entweder ohne Isolierung in Lösung oder nach Isolierung mit Salzsäure in einem Lösungsmittel (z.B. Dioxan) umsetzt.

Ein weiterer Gegenstand der vorliegenden Erfindung ist des Alaninat-Dihydrochlorid der Formel (VIII)

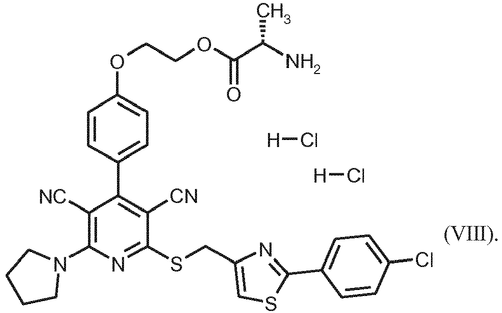

Ein weiterer Gegenstand der vorliegenden Erfindung ist ein Verfahren zur Herstellung des Alaninat-Dihydrochlorids der Formel (VIII), dadurch gekennzeichnet, dass man die Verbindung der Formel (IV) mit Boc-L-Alanin in Gegenwart eines Kondensationsmittel (z.B. Dicyclohexylcarbodiimid) und in Gegewart von 4-(Dimethylamino)-pyridin in einem Lösungsmittel (z.B. Tetrahydrofuran) umsetzt und das so erhaltene geschützte Boc-Dialaninat (VII) entweder ohne Isolierung in Lösung oder nach Isolierung mit Salzsäure in einem Lösungsmittel (z.B. Tetrahydrofuran) umsetzt.

Die Verbindung der Formel (I) wird in der Regel mikronisiert und in der Pharmazie formuliert. Es zeigt sich, dass die Verbindung der Formel (I) in kristalliner Form der Modifikation I sehr gute Stabilitäts-Eigenschaften (auch bei hoher Luftfeuchtigkeit) besitzt und über > 2 Jahre hinweg problemlos gelagert werden kann.

Mit der neuen erfinderischen Synthese ist es gelungen in sehr effizienter Weise die Verbindung der Formel (I) herzustellen. Das Verfahren bietet gegenüber dem Stand der Technik erhebliche Vorteile, was die Skalierbarkeit und technische Durchführung betrifft. Die Gesamtausbeute ist verglichen mit publizierten Daten signifikant höher, außerdem werden exzellente Reinheiten beim Wirkstoff erzielt. Das neue Verfahren ermöglicht die reproduzierbare, ökonomische Herstellung der definierten Verbindung der Formel (I) in kristalliner Form der Modifikation I, dessen Existenz im Stand der Technik bisher nicht beschrieben wurde.

Überraschenderweise wurden drei weitere Modifikationen und ein Isopropanol-Solvat der Verbindung der Formel (I) gefunden. Diese können erhalten werden, indem man die nach dem erfindungsgemäßen Verfahren erhaltene Verbindung der Formel (I) zunächst mikronisiert und dann wie folgt behandelt:
Durch Trocknung im Exsikkator über Phosphorpentoxid wurde Modifikation II mit einem Schmelzpunkt von 146 °C erhalten.

Modifikation III mit einem Umwandlungspunkt von 134 °C wurde erhalten, indem man mikronisiertes Material zuerst in Acetonitril suspendiert, dann bei Raumtemperatur für eine Woche rührt und dann bei Raumtemperatur stehen lässt, bis das Lösungsmittel verdampft ist.

Modifikation IV mit einem Schmelzpunkt von 122 °C wurde erhalten, indem man mikronisiertes Material zuerst in Methanol löst und dann bei Raumtemperatur stehen lässt, bis das Lösungsmittel verdampft ist.

Ein Isopropanol-Solvat wurde erhalten, indem man die Zwischenstufe Dialaninat-Dihydrochlorid der Formel (IX) bei Raumtemperatur mit Isopropanol/Wasser (98:2) rührt, den Feststoff isoliert und ohne Vakuum an der Luft bei Raumtemperatur trocknet.

Die erfindungsgemäßen Verbindungen, die Verbindung der Formel (I) und die Verbindung der Formel (I) in kristalliner Form der Modifikation I, wirken als partielle Adenosin A1 Rezeptor Agonisten und zeigen ein nicht vorhersehbares, wertvolles pharmakologisches Wirkspektrum. Sie eignen sich daher zur Verwendung als Arzneimittel zur Behandlung und/oder Prophylaxe von Krankheiten bei Menschen und Tieren.

Die erfindungsgemäßen Verbindungen sind allein oder in Kombination mit einem oder mehreren anderen Wirkstoffen zur Prävention und/oder Behandlung verschiedener Erkrankungen geeignet, so beispielsweise Erkrankungen des Herzkreislauf-Systems (kardiovaskulären Erkrankungen), zur Kardioprotektion nach Schädigungen des Herzens sowie von Stoffwechsel- und Nierenerkrankungen.

Im Sinne der vorliegenden Erfindung sind unter Erkrankungen des Herzkreislauf-Systems bzw. kardiovaskulären Erkrankungen beispielsweise die folgenden Erkrankungen zu verstehen: Hypertonie, periphere und kardiale Gefäßerkrankungen, koronare Herzerkrankung, koronare Restenose wie z.B. Restenose nach Ballondilatation von peripheren Blutgefäßen, Myokardinfarkt, akutes Koronarsyndrom, akutes Koronarsyndrom mit ST-Erhebung, akutes Koronarsyndrom ohne ST-Erhebung, stabile und instabile Angina pectoris, Herzmuskelschwäche, Prinzmetal Angina, persistierende ischämische Dysfunktion (hibernating Myokardium), vorübergehende post-ischämische Dysfunktion (stunned Myokardium), Herzinsuffizienz, Tachykardien, atriale Tachykardie, Arrhythmien, Vorhof- und Kammerflimmern, persistierendes Vorhofflimmern, permanentes Vorhofflimmern, Vorhofflimmern mit normaler linksventrikulärer Funktion, Vorhofflimmern mit eingeschränkter linksventrikulärer Funktion, Wolff-Parkinson-White Syndrom, periphere Durchblutungsstörungen, erhöhte Spiegel von Fibrinogen und von LDL geringer Dichte, sowie erhöhte Konzentrationen von Plasminogenaktivator-Inhibitor 1 (PAI-1), insbesondere koronare Herzerkrankung, akutes Koronarsyndrom, Angina pectoris, Herzinsuffizienz, Myokardinfarkt und Vorhofflimmern

Im Sinne der vorliegenden Erfindung umfasst der Begriff Herzinsuffizienz sowohl akute als auch chronische Erscheinungsformen der Herzinsuffizienz, wie auch spezifischere oder verwandte Krankheitsformen wie akute dekompensierte Herzinsuffizienz, Rechtsherzinsuffizienz, Linksherzinsuffizienz, Globalinsuffizienz, ischämische Kardiomyopathie, dilatative Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappen-insuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische sowie systolische Herzinsuffizienz und akute Phasen der Verschlechterung einer bestehenden chronischen Herzinsuffizienz (worsening heart failure).

Weiterhin eignen sich die erfindungsgemäßen Verbindungen auch zur Reduktion des von einem Infarkt betroffenen Myokardbereichs sowie zur Prävention von Sekundärinfarkten.

Des Weiteren sind die erfindungsgemäßen Verbindungen zur Prävention und/oder Behandlung von thromboembolischen Erkrankungen, Reperfusionsschäden nach Ischämie, mikro- und makrovaskulären Schädigungen (Vaskulitis), arteriellen sowie venösen Thrombosen, Ödemen, Ischämien wie Myokardinfarkt, Hirnschlag und transitorischen ischämischen Attacken, zur Kardioprotektion bei Koronararterien Bypass Operationen (CABG), primären PTCAs, PTCAs nach Thrombolyse, Rescue-PTCA, Herztransplantationen und Operationen am offenen Herzen, sowie zur Organprotektion bei Transplantationen, Bypass Operationen, Herzkatheteruntersuchungen und anderen operativen Eingriffen geeignet.

Weitere Indikationsgebiete, für die die erfindungsgemäßen Verbindungen eingesetzt werden können, sind beispielsweise die Prävention und/oder Behandlung von Erkrankungen des Urogenitalbereiches, wie z.B. Reizblase, erektile Dysfunktion und weibliche sexuelle Dysfunktion, daneben aber auch die Prävention und/oder Behandlung von inflammatorischen Erkrankungen, wie z.B. entzündliche Dermatosen (Psoriasis, Akne, Ekzeme, Neurodermitis, Dermatitis, Keratitis, Narbenbildung, Warzenbildung, Frostbeulen), von Erkrankungen des Zentralen Nervensystems und neurodegenerativen Störungen (Schlaganfall, Alzheimer'sche Krankheit, Parkinson'sche Krankheit, Demenz, Epilepsie, Depressionen, Multiple Sklerose), von Schmerzzuständen, Krebserkrankungen (Hautkrebs, Liposarcome, Karzinome des Magen-Darm-Traktes, der Leber, Bauchspeicheldrüse, Lunge, Niere, Harnleiter, Prostata und des Genitaltraktes) sowie von Übelkeit und Erbrechen in Verbindung mit Krebstherapien.

Weitere Indikationsgebiete sind beispielsweise die Prävention und/oder Behandlung von Entzündungs- und Immunerkrankungen (Morbus Crohn, Colitis ulcerosa, Lupus erythematodes, rheumatoide Arthritis) und von Erkrankungen der Atemwege, wie beispielsweise chronisch-obstruktive Atemwegserkrankungen (chronische Bronchitis, COPD), Asthma, Lungenemphysem, Bronchiektasien, zystische Fibrose (Mukoviszidose) und pulmonale Hypertonie, insbesondere pulmonale arterielle Hypertonie.

Schließlich kommen die erfindungsgemäßen Verbindungen auch für die Prävention und/ oder Behandlung von Diabetes, insbesondere Diabetes mellitus, Gestationsdiabetes, Insulin-abhängiger Diabetes und Nicht-Insulin-abhängiger Diabetes, von diabetischen Folgeerkrankungen wie z.B. Retinopathie, Nephropathie und Neuropathie, von metabolischen Erkrankungen (Metabolisches Syndrom, Hyperglykämie, Gestationsdiabetes, Hyperinsulinämie, Insulinresistenz, Glukose-Intoleranz, Fettsucht (Adipositas)) sowie von Arteriosklerose und Dyslipidämien (Hypercholesterolämie, Hypertriglyceridämie, erhöhte Konzentrationen der postprandialen Plasma-Triglyceride, Hypoalphalipoproteinämie, kombinierte Hyperlipidämien), insbesondere von Diabetes, Metabolischem Syndrom und Dyslipidämien, in Betracht

Darüber hinaus können die erfindungsgemäßen Verbindungen auch zur Behandlung und/oder Prävention von Schilddrüsenerkrankungen (Hyperthyreose), Erkrankungen der Bauchspeicheldrüse (Pankreatitis), Leberfibrose, viralen Erkrankungen (HPV, HCMV, HIV), Kachexie, Osteoporose, Gicht, Inkontinenz sowie zur Wundheilung und Angiogenese eingesetzt werden.

Weiterer Gegenstand der vorliegenden Erfindung ist die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Weiterer Gegenstand der vorliegenden Erfindung sind die erfindungsgemäßen Verbindungen zur Verwendung in einem Verfahren zur Behandlung und/oder Prophylaxe von koronarer Herzerkrankung, akutem Koronarsyndrom, Angina pectoris, Herzinsuffizienz, Myokardinfarkt und Vorhofflimmern.

Weiterer Gegenstand der vorliegenden Erfindung sind die erfindungsgemäßen Verbindungen zur Verfahren zur Behandlung und/oder Prophylaxe von Diabetes, Metabolischem Syndrom und Dyslipidämien.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen.

Als geeignete Kombinationswirkstoffe seien beispielhaft und vorzugsweise genannt: den Fettstoffwechsel verändernde Wirkstoffe Antidiabetika, Blutdruck-Senker, durchblutungsfördernd und/ oder antithrombotisch wirkende Mittel, Antioxidantien, Chemokin-Rezeptor-Antagonisten, p38-Kinase-Inhibitoren, NPY-Agonisten, Orexin-Agonisten, Anorektika, PAF-AH-Inhibitoren, Antiphlogistika (COX-Inhibitoren, LTB4-Rezeptor-Antagonisten), Analgetika wie beispielsweise Aspirin, Anti-Depressiva und andere Psychopharmaka.

Gegenstand der vorliegenden Erfindung sind insbesondere Kombinationen mindestens einer der erfindungsgemäßen Verbindungen mit mindestens einem den Fettstoffwechsel verändernden Wirkstoff, einem Antidiabetikum, einem blutdrucksenkenden Wirkstoff und/oder einem antithrombotisch wirkenden Mittel.

Die erfindungsgemäßen Verbindungen können vorzugsweise mit einem oder mehreren
- den Fettstoffwechsel verändernden Wirkstoffen, beispielhaft und vorzugsweise aus der Gruppe der HMG-CoA-Reduktase-Inhibitoren, Inhibitoren der HMG-CoA-Reduktase-Expression, Squalensynthese-Inhibitoren, ACAT-Inhibitoren, LDL-Rezeptor-Induktoren, Cholesterin-Absorptionshemmer, polymeren Gallensäureadsorber, Gallensäure-Reabsorptionshemmer, MTP-Inhibitoren, Lipase-Inhibitoren, LpL-Aktivatoren, Fibrate, Niacin, CETP-Inhibitoren, PPAR-α-, PPAR-γ- und/oder PPAR-δ-Agonisten, RXR-Modulatoren, FXR-Modulatoren, LXR-Modulatoren, Thyroidhormone und/oder Thyroidmimetika, ATP-Citrat-Lyase-Inhibitoren, Lp(a)-Antagonisten, Cannabinoid-Rezeptor 1-Antagonisten, Leptin-Rezeptor-Agonisten, Bombesin-Rezeptor-Agonisten, Histamin-Rezeptor-Agonisten sowie der Antioxidantien/Radikalfänger;
- Antidiabetika, die in der Roten Liste 2004/II, Kapitel 12 genannt sind, sowie beispielhaft und vorzugsweise jenen aus der Gruppe der Sulphonylharnstoffe, Biguanide, Meglitinid-Derivate, Glukosidase-Inhibitoren, Inhibitoren der Dipeptidyl-Peptidase IV (DPP-IV-Inhibitoren), Oxadiazolidinone, Thiazolidindione, GLP 1-Rezeptor-Agonisten, Glukagon-Antagonisten, Insulin-Sensitizer, CCK 1-Rezeptor-Agonisten, Leptin-Rezeptor-Agonisten, Inhibitoren von Leberenzymen, die an der Stimulation der Glukoneogenese und/ oder Glykogenolyse beteiligt sind, Modulatoren der Glukoseaufnahme sowie der Kaliumkanalöffner, wie z.B. denjenigen, die in WO 97/26265 und WO 99/03861 offenbart sind;
- den Blutdruck senkenden Wirkstoffen, beispielhaft und vorzugsweise aus der Gruppe der Calcium-Antagonisten, Angiotensin AII-Antagonisten, ACE-Hemmer, Renin-Inhibitoren, beta-Rezeptoren-Blocker, alpha-Rezeptoren-Blocker, Aldosteron-Antagonisten, Mineralocorticoid-Rezeptor-Antagonisten, ECE-Inhibitoren, ACE/NEP Inhibitoren sowie der Vasopeptidase-Inhibitoren; und/oder
- antithrombotisch wirkenden Mitteln, beispielhaft und vorzugsweise aus der Gruppe der Thrombozytenaggregationshemmer oder der Antikoagulantien
- Diuretika;
- Vasopressin-Rezeptor-Antagonisten;
- organischen Nitraten und NO-Donatoren;
- positiv-inotrop wirksamen Verbindungen;
- Verbindungen, die den Abbau von cyclischem Guanosinmonophosphat (cGMP) und/oder cyclischem A-denosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil sowie PDE 3-Inhibitoren wie Milrinone;
- natriuretischen Peptiden, wie z.B. "atrial natriuretic peptide" (ANP, Anaritide), "B-type natriuretic peptide" oder "brain natriuretic peptide" (BNP, Nesiritide), "C-type natriuretic peptide" (CNP) sowie Urodilatin;
- Agonisten des Prostacyclin-Rezeptors (IP-Rezeptors), wie beispielsweise Iloprost, Beraprost, Cicaprost;
- Hemmer des If (funny channel) Kanals, wie beispielsweise Ivabradine;
- Calcium-Sensitizern, wie beispielhaft und vorzugsweise Levosimendan;
- Kalium-Supplements;
- NO-unabhängigen, jedoch Häm-abhängigen Stimulatoren der Guanylatcyclase, wie insbesondere den in WO 00/06568, WO 00/06569, WO 02/42301 und WO 03/095451 beschriebenen Verbindungen;
- NO- und Häm-unabhängigen Aktivatoren der Guanylatcyclase, wie insbesondere den in WO 01/19355, WO 01/19776, WO 01/19778, WO 01/19780, WO 02/070462 und WO 02/070510 beschriebenen Verbindungen;
- Inhibitoren der humanen neutrophilen Elastase (HNE), wie beispielsweise Sivelestat und DX-890 (Reltran);
- die Signaltransduktionskaskade inhibierenden Verbindungen, wie beispielsweise Tyrosinkinase-Inhibitoren, insbesondere Sorafenib, Imatinib, Gefitinib und Erlotinib; und/oder
- den Energiestoffwechsel des Herzens beeinflussenden Verbindungen, wie beispielweise Etomoxir, Dichloracetat, Ranolazine und Trimetazidine kombiniert werden.

Unter den Fettstoffwechsel verändernden Wirkstoffen werden vorzugsweise Verbindungen aus der Gruppe der HMG-CoA-Reduktase-Inhibitoren, Squalensynthese-Inhibitoren, ACAT-Inhibitoren, Cholesterin-Absorptionshemmer, MTP-Inhibitoren, Lipase-Inhibitoren, Thyroidhormone und/oder Thyroidmimetika, Niacin-Rezeptor-Agonisten, CETP-Inhibitoren, PPAR-α-Agonisten, PPAR-γ-Agonisten, PPAR-δ-Agonisten, polymeren Gallensäureadsorber, Gallensäure-Reabsorptionshemmer, Antioxidantien/Radikalfänger sowie der Cannabinoid-Rezeptor 1-Antagonisten verstanden.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem Thyroidhormon und/oder Thyroidmimetikum, wie beispielhaft und vorzugsweise D-Thyroxin oder 3,5,3'-Triiodothyronin (T3), verabreicht.

Bei einer bevorzugten Ausführungsform der Erfindung werden die erfindungsgemäßen Verbindungen in Kombination mit einem Agonisten des Niacin-Rezeptors, wie beispielhaft und vorzugsweise Niacin, Acipimox, Acifran oder Radecol, verabreicht.

Bei einer bevorzugten Ausführungsform der Erfindung werden die erfindungsgemäßen Verbindungen in Kombination mit einem CETP-Inhibitor, wie beispielhaft und vorzugsweise Dalcetrapib, BAY 60-5521, Anacetrapib oder CETP-vaccine (CETi-1), verabreicht.

Bei einer bevorzugten Ausführungsform der Erfindung werden die erfindungsgemäßen Verbindungen in Kombination mit einem PPAR-γ-Agonisten beispielsweise aus der Klasse der Thiazolidindione, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-δ-Agonisten, wie beispielhaft und vorzugsweise GW-501516 oder BAY 68-5042, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Antioxidans/Radikalfänger, wie beispielhaft und vorzugsweise Probucol, AGI-1067, BO-653 oder AEOL-10150, verabreicht.

Bei einer bevorzugten Ausführungsform der Erfindung werden die erfindungsgemäßen Verbindungen in Kombination mit einem Cannabinoid-Rezeptor 1-Antagonisten, wie beispielhaft und vorzugsweise Rimonabant oder SR-147778, verabreicht.

Unter Antidiabetika werden vorzugsweise Insulin und Insulinderivate sowie oral wirksame hypoglykämische Wirkstoffe verstanden. Insulin und Insulinderivate umfasst hierbei sowohl Insuline tierischen, menschlichen oder biotechnologischen Ursprungs als auch Gemische hieraus. Die oral wirksamen hypoglykämischen Wirkstoffe umfassen vorzugsweise Sulphonylharnstoffe, Biguanide, Meglitinid-Derivate, Glukosidase-Inhibitoren und PPAR-gamma-Agonisten.

Bei einer bevorzugten Ausführungsform der Erfindung werden die erfindungsgemäßen Verbindungen in Kombination mit Insulin verabreicht.

Bei einer bevorzugten Ausführungsform der Erfindung werden die erfindungsgemäßen Verbindungen in Kombination mit einem Sulphonylharnstoff, wie beispielhaft und vorzugsweise Tolbutamid, Glibenclamid, Glimepirid, Glipizid oder Gliclazid, verabreicht.

Bei einer bevorzugten Ausführungsform der Erfindung werden die erfindungsgemäßen Verbindungen in Kombination mit einem Biguanid, wie beispielhaft und vorzugsweise Metformin, verabreicht.

Bei einer bevorzugten Ausführungsform der Erfindung werden die erfindungsgemäßen Verbindungen in Kombination mit einem Meglitinid-Derivat, wie beispielhaft und vorzugsweise Repaglinid oder Nateglinid, verabreicht.

Bei einer bevorzugten Ausführungsform der Erfindung werden die erfindungsgemäßen Verbindungen in Kombination mit einem Glukosidase-Inhibitor, wie beispielhaft und vorzugsweise Miglitol oder Acarbose, verabreicht.

Bei einer bevorzugten Ausführungsform der Erfindung werden die erfindungsgemäßen Verbindungen in Kombination mit einem DPP-IV-Inhibitor, wie beispielhaft und vorzugsweise Sitagliptin und Vildagliptin, verabreicht.

Bei einer bevorzugten Ausführungsform der Erfindung werden die erfindungsgemäßen Verbindungen in Kombination mit einem PPAR-gamma-Agonisten beispielsweise aus der Klasse der Thiazolindione, wie beispielhaft und vorzugsweise Pioglitazon und Rosiglitazon, verabreicht.

Unter den Blutdruck senkenden Mitteln werden vorzugsweise Verbindungen aus der Gruppe der Calcium-Antagonisten, Angiotensin AII-Antagonisten, ACE-Hemmer, beta-Rezeptoren-Blocker, alpha-Rezeptoren-Blocker und Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Valsartan, Candesartan, Embusartan, Olmesartan oder Telmisartan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem alpha-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Bei einer bevorzugten Ausführungsform der Erfindung werden die erfindungsgemäßen Verbindungen in Kombination mit einem Aldosteron- oder Mineralokortikoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Vasopressin-Rezeptor-Antagonisten, wie beispielhaft und vorzugsweise Conivaptan, Tolvaptan, Lixivaptan oder SR-121463, verabreicht.

Bei einer bevorzugten Ausführungsform der Erfindung werden die erfindungsgemäßen Verbindungen in Kombination mit einem organischen Nitrat oder NO-Donator, wie beispielhaft und vorzugsweise Natriumnitroprussid, Nitroglycerin, Isosorbidmononitrat, Isosorbiddinitrat, Molsidomin oder SIN-1, oder in Kombination mit inhalativem NO verabreicht.

Bei einer bevorzugten Ausführungsform der Erfindung werden die erfindungsgemäßen Verbindungen in Kombination mit einer positiv-inotrop wirksamen Verbindung, wie beispielhaft und vorzugsweise Herzglycosiden (Digoxin), beta-adrenergen und dopaminergen Agonisten wie Isoproterenol, Adrenalin, Noradrenalin, Dopamin oder Dobutamin, verabreicht.

Bei einer bevorzugten Ausführungsform der Erfindung werden die erfindungsgemäßen Verbindungen in Kombination mit Antisympathotonika wie Reserpin, Clonidin oder alpha-Methyl-Dopa, mit Kaliumkanal-Agonisten wie Minoxidil, Diazoxid, Dihydralazin oder Hydralazin, oder mit Stickoxid freisetzenden Stoffen wie Glycerinnitrat oder Nitroprussidnatrium verabreicht.

Unter antithrombotisch wirkenden Mitteln werden vorzugsweise Verbindungen aus der Gruppe der Thrombozytenaggregationshemmer oder der Antikoagulantien verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Dabigatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Besonders bevorzugt im Rahmen der vorliegenden Erfindung sind Kombinationen enthaltend mindestens eine der erfindungsgemäßen Verbindungen sowie einen oder mehrere weitere Wirkstoffe ausgewählt aus der Gruppe bestehend aus HMG-CoA-Reduktase-Inhibitoren (Statine), Diuretika, beta-Rezeptoren-Blocker, organische Nitrate und NO-Donatoren, ACE-Inhibitoren, Angiotensin AII-Antagonisten, Aldosteron- und Mineralokortikoid-Rezeptor-Antagonisten, Vasopressin-Rezeptor-Antagonisten, Thrombozytenaggregationshemmer und Antikoagulantien, sowie deren Verwendung zur Behandlung und/oder Prävention der zuvor genannten Erkrankungen.

Im Sinne der vorliegenden Erfindung ist die Eignung zur Behandlung und/oder Prophylaxe von akuten Nierenerkrankungen, insbesondere in der Eignung zur Behandlung und/oder Prophylaxe akuter Niereninsuffizienz, sowie von akutem Nierenversagen (Primärerkrankung und Sekundärerkrankung) zu verstehen.

Im Sinne der vorliegenden Erfindung ist die Eignung zur Behandlung und/oder Prophylaxe von chronischen Nierenerkrankungen, insbesondere in der Eignung zur Behandlung und/oder Prophylaxe chronischer Niereninsuffizienz, sowie von chronischem Nierenversagen (Primärerkrankung und Sekundärerkrankung) zu verstehen.

Im Sinne der vorliegenden Erfindung umfasst der Begriff akute Niereninsuffizienz akute Erscheinungsformen der Nierenerkrankung, des Nierenversagens und/oder der Niereninsuffizienz mit und ohne Dialysepflicht, wie auch zugrundeliegende oder verwandte Nierenerkrankungen wie renale Hypoperfusion, intradialytische Hypotonie, Volumenmangel (z.B. Dehydratation, Blutverlust), Schock, akute Glomerulonephritis, hämolytischurämisches Syndrom (HUS), vaskuläre Kathastrophe (arterielle oder venöse Thrombose oder Embolie), Cholesterinembolie, akute Bence-Jones-Niere bei Plasmozytom, akute supravesikal oder subvesikale Abflussbehinderungen, immunlogische Nierenerkrankungen wie Nierentransplantatabstoßung, Immunkomplex-induzierte Nierenerkrankungen, tubuläre Dilatation, Hyperphosphatämie und/oder akute Nierenerkrankungen, die durch die Notwendigkeit zur Dialyse charakterisiert werden können. Des Weiteren, bei Teilresektionen der Niere, Dehydratation durch forcierte Diurese, unkontrolliertem Blutdruckanstieg mit maligner Hypertonie, Harnwegsobstruktion und -infekt und Amyloidose sowie Systemerkrankungen mit glomerulärer Beteiligung, wie rheumatologisch-immunologische Systemerkrankungen, wie beispielsweise Lupus erythematodes, Nierenarterienthrombose, Nierenvenenthrombose, Analgetikanephropathie und renal-tubuläre Azidose, sowie Röntgen-Kontrastmittel- sowie Medikamenten-induzierte akute interstitielle Nierenerkrankungen.

Im Sinne der vorliegenden Erfindung umfasst der Begriff chronische Niereninsuffizienz chronische Erscheinungsformen der Nierenerkrankung, des Nierenversagens und/oder der Niereninsuffizienz mit und ohne Dialysepflicht, wie auch zugrundeliegende oder verwandte Nierenerkrankungen wie renale Hypoperfusion, intradialytische Hypotonie, obstruktive Uropathie, Glomerulopathien, glomeruläre und tubuläre Proteinurie, renale Ödeme, Hämaturie, primäre, sekundäre sowie chronische Glomerulonephritis, membranöse und membranoproliferative Glomerulonephritis, Alport-Syndrom, Glomerulosklerose, tubulointerstitielle Erkrankungen, nephropathische Erkrankungen wie primäre und angeborene Nierenerkrankung, Nierenentzündung, immunlogische Nierenerkrankungen wie Nierentransplantatabstoßung, Immunkomplex-induzierte Nierenerkrankungen, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Creatinin- und/oder Wasser-Ausscheidung, abnorm erhöhte Blutkonzentrationen von Harnstoff, Stickstoff, Kalium und/oder Creatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können., des Weiteren, chronische Niereninsuffizienz bei Nierenzellkarzinomen, nach Teilresektionen der Niere, Dehydratation durch forcierte Diurese, unkontrollierter Blutdruckanstieg mit maligner Hypertonie, Harnwegsobstruktion und -infekt und Amyloidose sowie Systemerkrankungen mit glomerulärer Beteiligung, wie rheumatologisch-immunologische Systemerkrankungen, wie beispielsweise Lupus erythematodes, sowie Nierenarterienstenose, Nierenarterienthrombose, Nierenvenenthrombose, Analgetikanephropathie und renal-tubuläre Azidose. Weiterhin chronische Niereninsuffizienz basierend auf Röntgen-Kontrastmittel- sowie Medikamenten-induzierte chronische interstitielle Nierenerkrankungen, Metabolisches Syndrom und Dyslipidämie. Die vorliegende Erfindung umfasst auch die Verwendung der erfindungsgemäßen Verbindungen zur Behandlung und/oder Prophylaxe von Folgeerscheinungen einer Niereninsuffizienz, wie beispielsweise Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### Experimenteller Teil

### Abkürzungen und Akronyme

- DCC: Dicyclohexylcarbodiimid
- DMAP: 4-(Dimethylamino)-pyridin
- g: Gramm
- h: Stunde
- HPLC: Hochdruckflüssigkeitschromatographie
- kg: Kilogramm
- L: Liter
- min: Minute
- MS: Masse aus Massenspektrometrie
- THF: Tetrahydrofuran

### Beispiele

### Beispiel 1

### 2-Amino-6-({[2-(4-chlorphenyl)-1,3-thiazol-4-yl]methyl}sulfanyl)-4-[4-(2-hydroxyethoxy)phenyl]pyridin-3,5-dicarbonitril (II)

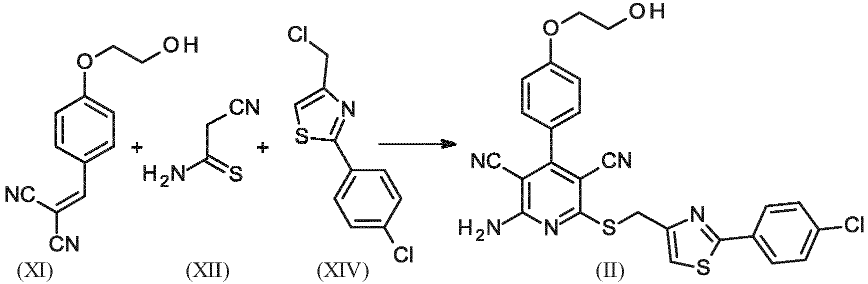

5,334 kg (24,90 mol) 2-[4-(2-Hydroxyethoxy)benzyliden]malononitril (XI) und 1,309 kg (13,07 mol) 2-Cyanthioacetamid (XII) wurden in 27,4 kg (34,8 L) Methanol suspendiert. Man erwärmte auf 40 °C und dosierte 3,779 kg (37,35 mol) Triethylamin bei max. 40 °C ein. Man rührte noch 3 h bei 40 °C nach und kühlte auf Raumtemperatur ab. Zu der dunkelbraunen Lösung gab man 3,147 kg (12,45 mol) an 4-(Chlormethyl)-2-(4-chlorphenyl)-1,3-thiazol (XIV) und rührte den Kesselinhalt über Nacht bei Raumtemperatur. Die nun vorliegende Suspension wurde auf 5 °C abgekühlt, durch Filtration isoliert und mit insgesamt 11,7 kg (14,85 L) Methanol gewaschen. Das Feuchtprodukt wurde bei 50 °C im Vakuumtrockenschrank getrocknet.

Man erhielt 4862 g bzw. 75,1 % der Theorie an 2-Amino-6-({[2-(4-chlorphenyl)-1,3-thiazol-4-yl]methyl}-sulfanyl)-4-[4-(2-hydroxyethoxy)phenyl]pyridin-3,5-dicarbonitril als beige-grünlichen Feststoff (Gehalt 95,5 %, ESTD).
HPLC-Methode A: Retentionszeit ca. 14,1 min.

### Beispiel 2

### 2-Chlor-6-({[2-(4-chlorphenyl)-1,3-thiazol-4-yl]methyl}sulfanyl)-4-[4-(2-hydroxyethoxy)phenyl]pyridin-3,5-dicarbonitril (III)

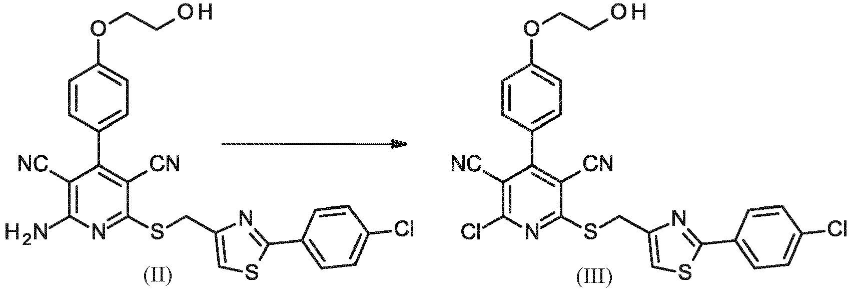

1,75 kg (7,68 mol) Benzyltriethylammoniumchlorid wurden in 14,0 kg (17,8 L) Acetonitril bei Raumtemperatur gelöst. Dazu gab man 2,0 kg (3,846 mol) an 2-Amino-6-({[2-(4-chlorphenyl)-1,3-thiazol-4-yl]-methyl}sulfanyl)-4-[4-(2-hydroxyethoxy)phenyl]pyridin-3,5-dicarbonitril (II). In die Suspension wurde dann 1,66 kg (12,35 mol) Kupfer(II)chlorid gegeben, mit 1,00 kg (1,27 L) Acetonitril nachgespült und auf 50 °C erwärmt. Bei dieser Temperatur wurde 793 g (7,69 mol) an tert-Butylnitrit eindosiert und mit 1,00 kg (1,27 L) Acetonitril nachgespült. Der Kesselinhalt wurde noch 3 h bei 50 °C gerührt, auf Raumtemperatur abgekühlt und mit 9,0 kg (10,0 L) Essigsäureethylester und 10 kg (10 L) Wasser verrührt. Nach dem Absitzen wurde die wässrige Phase abgetrennt und erneut mit 10 kg (10 L) Wasser verrührt. Nach erneutem Absitzen wurde die wässrige Phase abgetrennt. Die organische Phase wurde ein drittes Mal mit 10 kg (10 L) Wasser gewaschen, wobei zusätzlich weitere 3,6 kg (4,0 L) Essigsäureethylester zugegeben wurden. Aus der organischen Phase wurden bei 300 mbar und bis 42 °C maximaler Innentemperatur 15,5 L Destillat abgenommen. Zu der zurückbleibenden Suspension wurden 9,6 kg (12,1 L) Methanol gegeben und der Kesselinhalt wurde 1 h bei Rückfluss (ca. 67 °C) gerührt. Die resultierende Suspension wurde abgesaugt und mit insgesamt 8 kg (10,1 L) Methanol gewaschen. Das Feuchtprodukt wurde über Nacht bei 50 °C im Vakuumtrockenschrank konstant getrocknet.

Man erhielt 1636 g bzw. 78,8 % der Theorie an 2-Chlor-6-({[2-(4-chlorphenyl)-1,3-thiazol-4-yl]methyl}-sulfanyl)-4-[4-(2-hydroxyethoxy)phenyl]pyridin-3,5-dicarbonitril als ockerfarbenes Pulver.

### Beispiel 3

### 2-({[2-(4-Chlorphenyl)-1,3-thiazol-4-yl]methyl}sulfanyl)-4-[4-(2-hydroxyethoxy)phenyl]-6-(pyrrolidin-1-yl)-pyridin-3,5-dicarbonitril (IV)

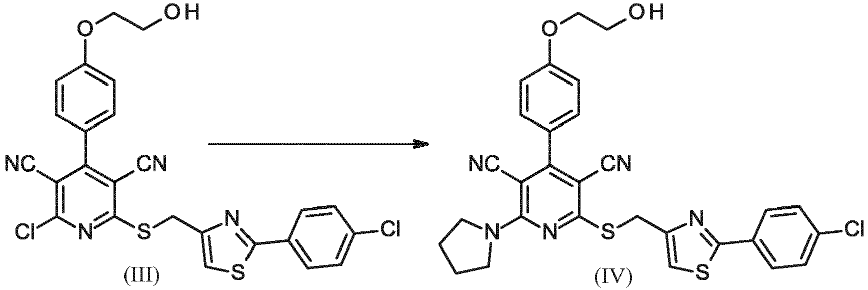

1,60 kg (2,966 mol) 2-Chlor-6-({[2-(4-chlorphenyl)-1,3-thiazol-4-yl]methyl}sulfanyl)-4-[4-(2-hydroxy-ethoxy)phenyl]pyridin-3,5-dicarbonitril (III) wurden in 27,2 kg (34,4 L) Methanol suspendiert. Dazu dosierte man innerhalb von 30 min 1,58 kg (22,24 mol) Pyrrolidin so zu, dass die Innentemperatur von 30 °C nicht überschritten wurde. Dann wurde über Nacht bei Raumtemperatur und dann für 1 h bei Rückfluss (65-66 °C) gerührt. Man kühlte auf Raumtemperatur ab, filtrierte den Feststoff ab und ab und wusch mit insgesamt 7,6 kg (9,6 L) Methanol nach. Das Feuchtprodukt wurde bei 50 °C im Vakuum getrocknet.

Man erhielt 1549 g bzw. 91,0 % der Theorie an 2-({[2-(4-Chlorphenyl)-1,3-thiazol-4-yl]methyl}sulfanyl)-4-[4-(2-hydroxyethoxy)phenyl]-6-(pyrrolidin-1-yl)pyridin-3,5-dicarbonitril als beiges Pulver.
HPLC-Methode B: Retentionszeit ca. 12,0 min.

Als Nebenkomponenten werden typischerweise erhalten:
N-{6-({[2-(4-Chlorphenyl)-1,3-thiazol-4-yl]methyl}sulfanyl)-3,5-dicyano-4-[4-(hydroxyethoxy)phenyl]-pyridin-2-yl}acetamid ("Aminoacetylmercaptothiazolo-pyridin") mit einem typischen Gehalt von 0,2 %. HPLC-Methode B: relative Retentionszeit 0,56 (ca. 7,3 min.).
2-Amino-6-({[2-(4-chlorphenyl)-1,3-thiazol-4-yl]methyl}sulfanyl)-4-[4-(2-hydroxyethoxy)phenyl]pyridin-3,5-dicarbonitril (II) mit einem typischen Gehalt von 0,3 %. HPLC-Methode B: relative Retentionszeit 0,62 (ca. 8,1 min.).
2-({[2-(4-Chlorphenyl)-1,3-thiazol-4-yl]methyl}sulfanyl)-6-hydroxy-4-[4-(2-hydroxyethoxy)phenyl]-pyridin-3,5-dicarbonitril ("Hydroxymercapthiazolopyridin") mit einem typischen Gehalt von 0,3 %. HPLC-Methode B: relative Retentionszeit 1,32 (ca. 17,3 min.).

### Beispiel 4

### 2-({[2-(4-Chlorphenyl)-1,3-thiazol-4-yl]methyl}sulfanyl)-4-[4-(2-hydroxyethoxy)phenyl]-6-(pyrrolidin-1-yl)-pyridin-3,5-dicarbonitril (IV)

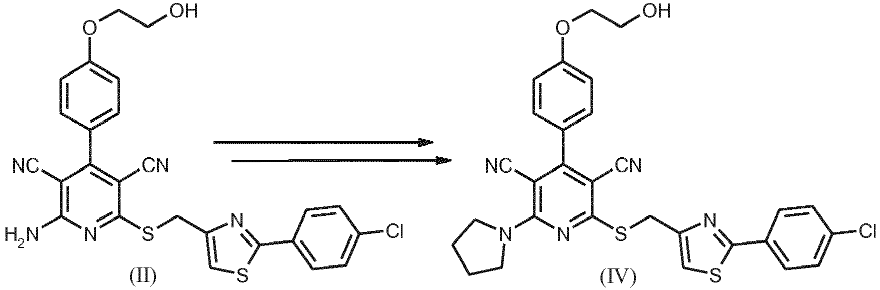

### 1. Teilschritt

1,23 kg (5,38 mol) Benzyltriethylammoniumchlorid wurden bei Raumtemperatur in 10,9 kg (13,9 L) Acetonitril gelöst. Dazu gab man 1,40 kg (2,69 mol) an 2-Amino-6-({[2-(4-chlorphenyl)-1,3-thiazol-4-yl]-methyl}sulfanyl)-4-[4-(2-hydroxyethoxy)phenyl]pyridin-3,5-dicarbonitril (II). In die Suspension wurde dann 1,16 kg (8,62 mol) Kupfer(II)chlorid gegeben, mit 0,42 kg (0,523 L) Acetonitril nachgespült und auf 50 °C erwärmt. Bei dieser Temperatur wurde 0,56 kg (5,38 mol) tert-Butylnitrit (ca.90%ig, Gehalt bei Berechnungen nicht berücksichtigt) eindosiert und mit 0,28 kg (0,36 L) Acetonitril nachgespült. Der Kesselinhalt wurde noch 4 h bei 50 °C gerührt. Es wurde auf Raumtemperatur abgekühlt und mit 17,0 kg (19,6 L) Isopropylacetat und 7,25 kg (13,9 mol) 7 % Salzsäure verrührt. Nach dem Absitzen wurde die wässrige Phase abgetrennt und erneut mit 7,25 kg (13,9 mol) 7 % Salzsäure verrührt. Nach erneutem Absitzen (10 min) wurde die wässrige Phase abgetrennt. Die organische Phase wurde ein drittes Mal mit 8,4 kg (7,0 L) gesättigter Kochsalzlösung verrührt. Danach wurden die Phasen getrennt und die wässrige Unterphase abgetrennt. Die hellbraune organische Oberphase wurde über eine Filterplatte von ausgefallenen Feinanteilen filtriert. Dabei wurde mit 0,54 kg (0,62 L) Isopropylacetat gespült und nachgewaschen. Die so erhaltene Lösung wurde ohne weitere Behandlung in die nächste Stufe eingesetzt.

Die Auswaage an Lösung betrug 27,34 kg. Auf den Einsatz von 2,69 mol bezogen waren rechnerisch 1,45 kg 2-Chlor-6-({[2-(4-chlorphenyl)-1,3-thiazol-4-yl]methyl}sulfanyl)-4-[4-(2-hydroxyethoxy)phenyl]pyridin-3,5-dicarbonitril in der Lösung enthalten.

### 2. Teilschritt

Zu der im ersten Teilschritt erzeugten Lösung von 27,34 kg (2,69 mol) 2-Chlor-6-({[2-(4-chlorphenyl)-1,3-thiazol-4-yl]methyl}sulfanyl)-4-[4-(2-hydroxyethoxy)phenyl]pyridin-3,5-dicarbonitril in Isopropylacetat gab man 5,51 kg (7,01 L) Methanol und dosierte 0,77 kg (10,77 mol) Pyrrolidin zu. Die Zugabe wurde durch Kühlung bei max. 25 °C gehalten. Man rührte noch 2 h bei Raumtemperatur nach und erhitzte über Nacht auf Rückfluss (65-66 °C). Man kühlte auf ca. 20 °C ab, rührte 2 h bei ca. 20 °C nach, kühlte weiter auf ca. 5 °C ab und rührte 1 h nach. Danach saugte man ab, spülte und wusch mit insgesamt 5,34 kg (6,8 L) Methanol. Das Feuchtprodukt (1,42 kg) wurde bei 50 °C im Vakuum getrocknet.

Man erhielt 1300 g (2,26 mol) bzw. 84,1 % der Theorie über beide Stufen an 2-({[2-(4-Chlorphenyl)-1,3-thiazol-4-yl]methyl}sulfanyl)-4-[4-(2-hydroxyethoxy)phenyl]-6-(pyrrolidin-1-yl)pyridin-3,5-dicarbonitril als beige-gelbes Pulver.

### Beispiel 5

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-alaninat-Dihydrochlorid (VIII)

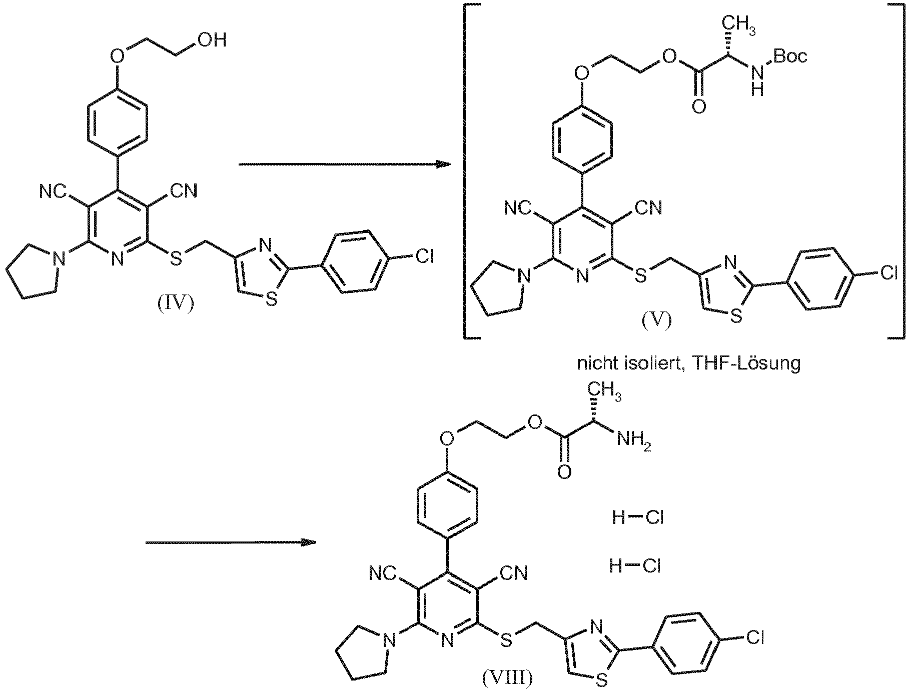

### 1. Teilschritt

1,250 kg (2,177 mol) 2-({[2-(4-Chlorphenyl)-1,3-thiazol-4-yl]methyl}sulfanyl)-4-[4-(2-hydroxyethoxy)phenyl]-6-(pyrrolidin-1-yl)pyridin-3,5-dicarbonitril (IV), 577 g (3,048 mol) Boc-L-Alanin und 8,25 kg (9,4 L) THF vorgelegt. Zu der Suspension gab man 133 g (1,093 mol) DMAP und dosierte dann eine Lösung aus 674 g (3,268 mol) DCC und 2,52 kg (2,9 L) THF bei ca. 20 °C ein. Der Kesselinhalt wurde über Nacht bei Raumtemperatur gerührt. Der ausgefallene Dicyclohexylharnstoff wurde abgesaugt und mit insgesamt 2,14 kg (2,4 L) THF gewaschen. Das resultierende Filtrat von ca. 14,5 kg wurde im Vakuum auf eine Menge von 3,2 kg eingeengt. Diese Lösung des Boc-L-Alaninats (V) wird ohne weitere Behandlung in die nächste Stufe eingesetzt.

### 2. Teilschritt

3,2 kg (2,177 mol) an Boc-L-Alaninat (V) Lösung aus dem 1. Teilschritt wurde mit 11,9 kg THF verdünnt. Dann wurde 6,90 kg (26,15 mol) einer Lösung von 4 M HCl in Dioxan bei ca. 23 °C (20-25 °C) Innentemperatur eindosiert. Schließlich wurde der Kesselinhalt 12 h bei ca. 23 °C (20-25 °C) gerührt. Der ausgefallene Feststoff wurde abgesaugt, mit insgesamt 19,0 kg (21,0 L) THF gewaschen. Das Feuchtprodukt wurde bei 40 °C im Vakuum in 20 h getrocknet.

Man erhielt eine Auswaage von 1719 g über beide Stufen an 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-alaninat-Dihydrochlorid. Berechnet mit dem Molgewicht von 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-alaninat-Dihydrochlorid ergab dies eine formale Ausbeute von 109 % der Theorie. Die formal über zu 100 % berechnete Ausbeute ist durch Reste an Reagenzien und Lösungsmitteln sowie im Wesentlichen durch Dimethylaminopyridin Hydrochlorid, das im isolierten Produkt enthalten ist, bedingt. Diese Verunreinigungen stellen keine Qualitätsminderung der Zwischenstufe Alaninat-Dihydrochlorid (VIII) dar, weil sämtliche Reagenzien in der nächsten Stufe erneut eingesetzt werden und die Folgestufen Boc-Dialaninat (VII) bzw. Dialaninat-Dihydrochlorid (IX) auf diese Verunreinigungen komplett gereinigt werden.
HPLC-Methode C: Retentionszeit ca. 13,1 min.

Als Nebenkomponenten werden typischerweise erhalten:
N,N-dimethylpyridin-4-amine (Dimethylaminopyridine) mit einem typischen Gehalt von 8 bis 10 %. HPLC-Methode C: relative Retentionszeit 0,15 (ca. 1,8 min.).
2-{4-[2-({[2-(4-Chlorphenyl)-1,3-thiazol-4-yl]methyl}sulfanyl)-3,5-dicyano-6-hydroxypyridin-4-yl]-phenoxy}ethyl L-alaninat ("Hydroxy-alaninat") mit einem typischen Gehalt von 0,3 %. HPLC-Methode C: relative Retentionszeit 0,77 (ca. 9,2 min.).
2-({[2-(4-Chlorphenyl)-1,3-thiazol-4-yl]methyl}sulfanyl)-4-[4-(2-hydroxyethoxy)phenyl]-6-(pyrrolidin-1-yl)pyridin-3,5-dicarbonitril (IV) mit einem typischen Gehalt von 0,5 bis 1 %.
   HPLC-Methode C: relative Retentionszeit 1,71 (ca. 20,5 min.).

### Beispiel 6

### 2-{4-[2-({[2-(4-Chlorphenyl)-1,3-thiazol-4-yl]methyl}sulfanyl)-3,5-dicyan-6-(pyrrolidin-1-yl)pyridin-4-yl]-phenoxy}ethyl-N-(tert-butoxycarbonyl)-L-alanyl-L-alaninat (VII)

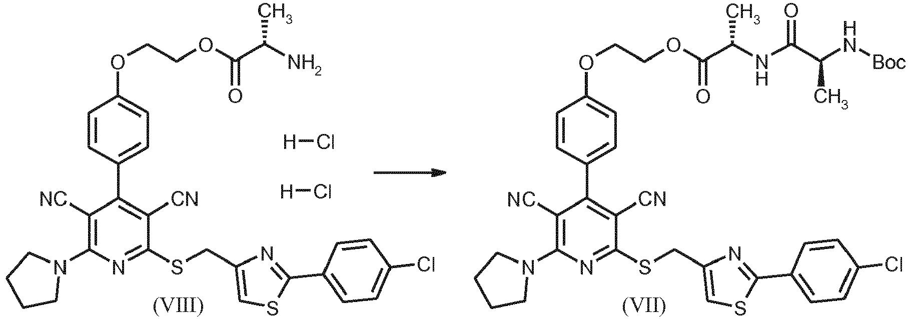

1,650 kg (2,298 mol) 2-{4-[2-({[2-(4-Chlorphenyl)-1,3-thiazol-4-yl]methyl}sulfanyl)-3,5-dicyan-6-(pyrrolidin-1-yl)pyridin-4-yl]phenxy}ethyl-L-alaninat-Dihydrochlorid (VIII), 14,7 kg THF und 562 g (4,599 mol) DMAP wurden vorgelegt und bei 20-25 °C gerührt. Dann gab man 522 g (2,757 mol) Boc-L-Alanin bei 20-25 °C zu. Dann wurde eine Lösung aus 570 g (2,761 mol) DCC und 3,10 kg THF zudosiert, mit 200 g THF nachgespült und 4 h bei 20-25 °C gerührt. Der ausgefallene Harnstoff wurde abfiltriert, wobei mit insgesamt 2,60 kg THF nachgewaschen wurde. Die resultierende Lösung wurde dreimal mit je 5,2 kg einer wässrigen, 15%igen Lösung von Ammoniumchlorid in Wasser gewaschen. Aus der wasserfeuchten organischen Phase wurde bei 200 mbar und max. 50 °C Innentemperatur eine Menge von 9,4 kg Destillat abgenommen. Zu dem zurückbleibenden Sumpf gab man 7,7 kg Dioxan zu und nahm erneut bei 200 mbar bis max. 60 °C Innentemperatur eine Menge von 10,2 kg Destillat ab. Zu dem Sumpf gab man erneut 7,7 kg Dioxan zu und nahm bei 200 mbar bis max. 70 °C Innentemperatur eine Menge von 7,9 kg Destillat ab. Zu dem Sumpf gab man ein drittes Mal 7,7 kg Dioxan zu und nahm bei 200 mbar bis max. 70 °C Innentemperatur eine letzte Menge von 7,9 kg Destillat ab. Schließlich gab man in Sumpf 6,7 kg Dioxan zu, filtrierte den Kesselinhalt zur Entfernung geringer Mengen von Feststoffen ab.

Das Filtrat wurde bei 20-25 °C in eine vorgelegte Menge von 22,3 kg Diisopropylether unter Rühren eindosiert. Man spülte mit 1,1 kg Dioxan nach. Der ausgefallen Feststoff wurde bei 20-25 °C filtriert. Man wusch mit 8,4 kg Diisopropylether nach. Der Feuchtkuchen wurde mit 5,2 kg Ethanol bei Raumtemperatur ca. 1 h ausgerührt, dann filtriert und mit 4,9 kg Ethanol gewaschen. Das Feuchtprodukt wurde im Trockenschrank bei 50 °C Manteltemperatur im Vakuum getrocknet.

Man erhielt an dieser Stelle 1403 g. Das entsprach ohne Gehaltsberücksichtigung des eingesetzten Alaninat Dihyhdrochlorides (VIII) einer Ausbeute von 74,8% der Theorie an 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-N-(tert-butoxycarbonyl)-L-alanyl-L-alaninat.
HPLC-Methode D: Retentionszeit ca. 11,9 min.

Als Nebenkomponenten werden typischerweise erhalten:
2-{4-[2-({[2-(4-Chlorphenyl)-1,3-thiazol-4-yl]methyl}sulfanyl)-3,5-dicyano-6-(pyrrolidin-1-yl)pyridin-4-yl]phenoxy}ethyl N-(tert-butoxycarbonyl)-L-alanyl-L-alanyl-L-alaninat ("Boc-Trialaninat") mit einem typischen Gehalt von 0,4 %.
   HPLC-Methode D: relative Retentionszeit 0,89 (ca. 10,6 min.).
2-{4-[2-({[2-(4-Chlorphenyl)-1,3-thiazol-4-yl]methyl}sulfanyl)-3,5-dicyano-6-(pyrrolidin-1-yl)pyridin-4-yl]phenoxy}ethyl N-(tert-butoxycarbonyl)-L-alaninat ("Boc-Alaninate", V) mit einem typischen Gehalt von 0,3 %.
   HPLC-Methode D: relative Retentionszeit 1,19 (ca. 14,1 min.).

### Beispiel 7

### 2-{4-[2-({[2-(4-Chlorphenyl)-1,3-thiazol-4-yl]methyl}sulfanyl)-3,5-dicyan-6-(pyrrolidin-1-yl)pyridin-4-yl]-phenoxy}ethyl-L-alanyl-L-alaninat-Dihydrochlorid (IX)

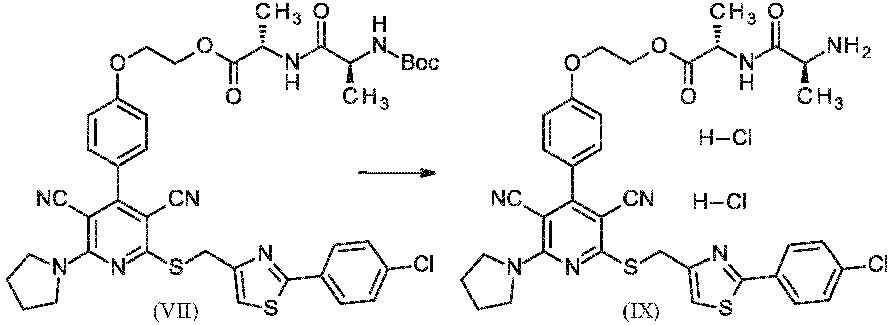

1440 g (1,76 mol) 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-N-(tert-butoxycarbonyl)-L-alanyl-L-alaninat (VII) wurden vorgelegt. Dazu gab man 26,0 kg Dichlormethan und erwärmte unter Rühren auf 40 °C. Die leicht getrübte Lösung wurde noch warm filtriert und mit 2,0 kg Dichlormethan nachgespült. Nun wurde bei Normaldruck ca. 16,6 kg (ca. 12,5 L) Destillat mit einer Vorlauftemperatur von maximal 60 °C abgenommen. Der zurückbleibende Sumpf wurde mit 3,5 kg Diisopropylether verdünnt. Dazu dosierte man 5,56 kg (21,2 mol) 4 M HCl in Dioxan bei 20-25 °C zu, spülte mit 200 g Dioxan nach und rührte bei Raumtemperatur über Nacht (16 h) nach. Der ausgefallene farblose Feststoff wurde abgesaugt und mit 18,6 kg Dichlormethan in drei gleich großen Portionen gewaschen. Anschließend wurde der Kuchen mit 13,5 kg Isopropanol gewaschen. Dann wurde der feuchte Kuchen entnommen und für 6 h in einem Vakuumtrockenschrank bei 50 °C getrocknet.

Man erhielt 1349 g bzw. 96,9% der Theorie an 2-{4-[2-({[2-(4-Chlorphenyl)-1,3-thiazol-4-yl]methyl}-sulfanyl)-3,5-dicyan-6-(pyrrolidin-1-yl)pyridin-4-yl]phenoxy}ethyl-L-alanyl-L-alaninat-Dihydrochlorid.

### Beispiel 8

### 2-{4-[2-({[2-(4-Chlorphenyl)-1,3-thiazol-4-yl]methyl}sulfanyl)-3,5-dicyan-6-(pyrrolidin-1-yl)pyridin-4-yl]-phenoxy}ethyl-L-alanyl-L-alaninat-Monohydrochlorid (I)

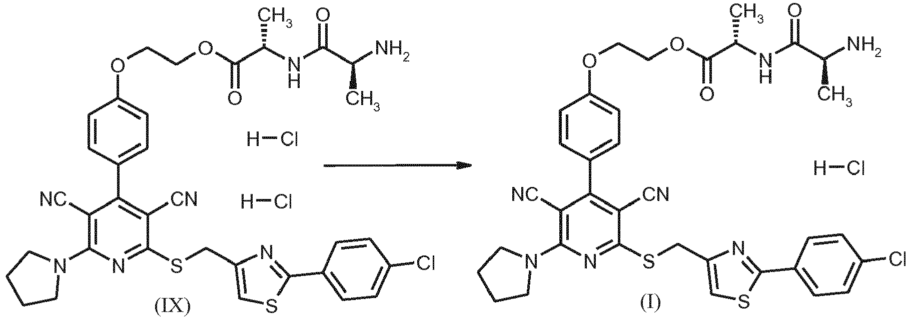

Der gesamte Auswaage von 1349 g an getrocknetem 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-alanyl-L-alaninat-Dihydrochlorid (IX) wurde in 21 kg Isopropanol/Wasser (98:2 v/v) für 67 h bei Raumtemperatur gerührt, dann filtriert und mit 9,6 kg Isopropanol in gewaschen. Das Produkt wurde im Vakuumtrockenschrank bei 50 °C in 16 h konstant getrocknet.

Man erhielt 1185 g (1,57 mol) bzw. 88,2 % der Theorie [bezogen auf den Einsatz des geschützten Boc-Dialaninats (VII)] bzw. 91,8 % der Theorie [bezogen auf das Dialaninat-Dihydrochlorid (IX)] an 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-alanyl-L-alaninat-Monohydrochlorid als farbloses Pulver.
HPLC-Methode E: Retentionszeit ca. 15,1 min.

Als Nebenkomponenten werden typischerweise erhalten:
2-{4-[2-({[2-(4-Chlorphenyl)-1,3-thiazol-4-yl]methyl}sulfanyl)-3,5-dicyano-6-(pyrrolidin-1-yl)pyridin-4-yl]phenoxy}ethyl L-alanyl-L-alanyl-L-alaninat ("Trialaninate") mit einem typischen Gehalt von 0,4 %. HPLC-Methode E: relative Retentionszeit 0,88 (ca. 13,3 min.).
rac 2-{4-[2-({[2-(4-Chlorphenyl)-1,3-thiazol-4-yl]methyl}sulfanyl)-3,5-dicyano-6-(pyrrolidin-1-yl)pyridin-4-yl]phenoxy}ethyl L-alanyl-D-alaninat ("D,L-Dialaninate") mit einem typischen Gehalt von 0,1 %. HPLC-Methode E: relative Retentionszeit 1,07 (ca. 16,2 min.).
2-{4-[2-({[2-(4-Chlorphenyl)-1,3-thiazol-4-yl]methyl}sulfanyl)-3,5-dicyano-6-(pyrrolidin-1-yl)pyridin-4-yl]phenoxy}ethyl L-alaninat ("Alaninat") mit einem typischen Gehalt von 0,3 %.
   HPLC-Methode E: relative Retentionszeit 1,35 (ca. 20,4 min.).
2-({[2-(4-Chlorphenyl)-1,3-thiazol-4-yl]methyl}sulfanyl)-4-[4-(2-hydroxyethoxy)phenyl]-6-(pyrrolidin-1-yl)-pyridin-3,5-dicarbonitril (V) mit einem typischen Gehalt von 0,3 %.
   HPLC-Methode E: relative Retentionszeit 1,60 (ca. 24,1 min.).
2-{4-[2-({[2-(4-Chlorphenyl)-1,3-thiazol-4-yl]methyl}sulfanyl)-3,5-dicyano-6-(pyrrolidin-1-yl)pyridin-4-yl]phenoxy}ethyl N-(tert-butoxycarbonyl)-L-alanyl-L-alaninat ("Boc-Dialaninate") mit einem typischen Gehalt von 0,1 %.
   HPLC-Methode E: relative Retentionszeit 2,35 (ca. 35,5 min.).

### Beispiel 9

### 2-{4-[2-({[2-(4-Chlorphenyl)-1,3-thiazol-4-yl]methyl}sulfanyl)-3,5-dicyan-6-(pyrrolidin-1-yl)pyridin-4-yl]-phenoxy}ethyl-L-alanyl-L-alaninat-Dihydrochlorid (IX)

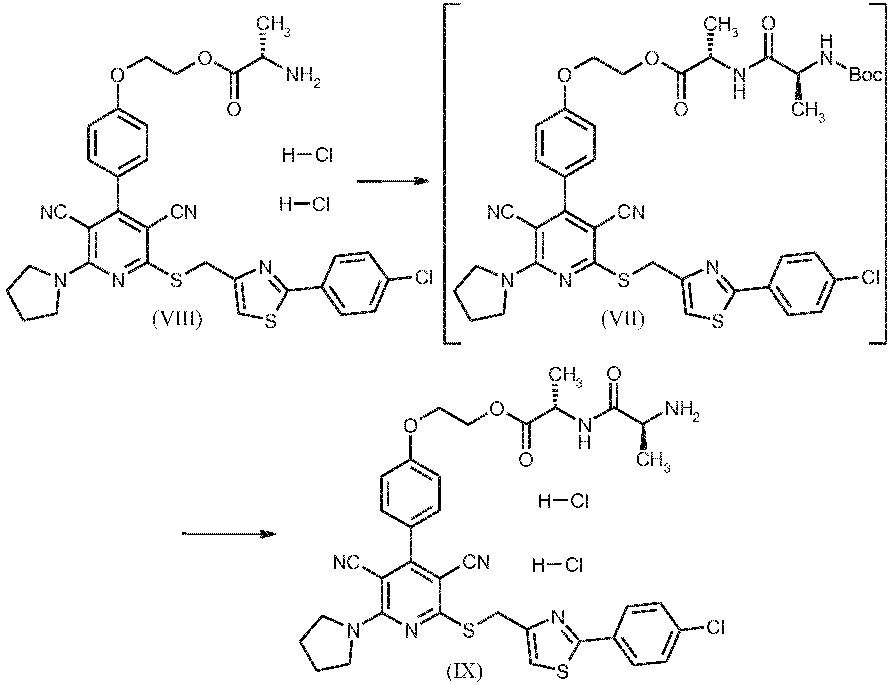

### 1. Teilschritt

1,37 kg (1,908 mol) 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-alaninat-Dihydrochlorid (VIII), 12,2 kg THF und 466 g (3,813 mol) DMAP wurden vorgelegt bei 20-25 °C verrührt. Dann gab man 432 g (2,281 mol) Boc-L-Alanin bei 20-25 °C zu. Dann wurde eine Lösung aus 473 g (2,291 mol) DCC und 2,33 kg THF zudosiert, mit 400 g THF nachgespült und 4 h bei 20-25 °C gerührt. Der ausgefallene Harnstoff wurde abfiltriert, wobei mit insgesamt 2,40 kg THF nachgespült wurde. Die resultierende Lösung wurde dreimal mit je 4,3 kg einer wässrigen, 15%-igen Lösung von Ammoniumchlorid in Wasser gewaschen. Aus der organischen Phase wurde bei 200 mbar und max. 50 °C Innentemperatur eine Menge von 8,2 kg Destillat abgenommen. Zu dem zurückbleibenden Sumpf gab man 6,9 kg Dioxan zu und nahm erneut bei 200 mbar bis max. 60 °C Innentemperatur eine Menge von 9,6 kg Destillat ab. Zu dem Sumpf gab man erneut 6,9 kg Dioxan zu und nahm bei 200 mbar bis max. 70 °C Innentemperatur eine Menge von 6,9 kg Destillat ab. Zu dem Sumpf gab man ein drittes Mal 6,9 kg Dioxan zu und nahm bei 200 mbar bis max. 70 °C Innentemperatur eine letzte Menge von 6,2 kg Destillat ab. Schließlich gab man in den Sumpf 19,0 kg Dioxan zu, filtrierte den Kesselinhalt zur Entfernung geringer Mengen von Feststoffen und spülte dabei mit 3,6 kg Dioxan nach.

Die resultierende Lösung (ca. 24,2 kg) wurde in dieser Form für den 2. Teilschritt eingesetzt.

### 2. Teilschritt

24,2 kg (1,908 mol) der Lösung aus dem 1. Teilschritt wurden vorgelegt und bei 20 °C gerührt. Dazu dosierte man 6,01 kg (22,89 mol) einer ca. 4,0 M Lösung von HCl in Dioxan innerhalb von bei ca. 20 °C Innentemperatur ein und rührte über Nacht bei Raumtemperatur nach. Dann wurde der Kesselinhalt filtriert, mit insgesamt 10,1 kg Dioxan nachgespült und gewaschen und bei 50 °C im Vakuum getrocknet.

### Beispiel 10

### 2-{4-[2-({[2-(4-Chlorphenyl)-1,3-thiazol-4-yl]methyl}sulfanyl)-3,5-dicyan-6-(pyrrolidin-1-yl)pyridin-4-yl]-phenoxy}ethyl-L-alanyl-L-alaninat-Hydrochlorid-Nachstellung von Beispiel 44 aus WO 2010/086101

1.5 g (1.837 mmol) des geschützten Boc-Dialaninats (VII) wurden in 24 ml Dichlormethan vorgelegt. Man heizte kurz auf Rückfluss, ließ auf Raumtemperatur abkühlen und rührte nach Versetzen mit 18.37 ml einer IN Lösung von Chlorwasserstoff in Diethylether über Nacht bei Raumtemperatur. Der entstandene Feststoff wurde abgesaugt und 2-mal mit je ca. 20 mL Diethylether gewaschen. Nach Trocknung über Nacht unter Vakuum bei 50°C wurden 1.43 g der Zielverbindung erhalten.

Die Chlorid-Bestimmung ergab einen Gehalt von 7,8 Gewichtsprozent, was in etwa 1,7 mol HCl pro Mol des heterocyclischen Grundkörpers entspricht.

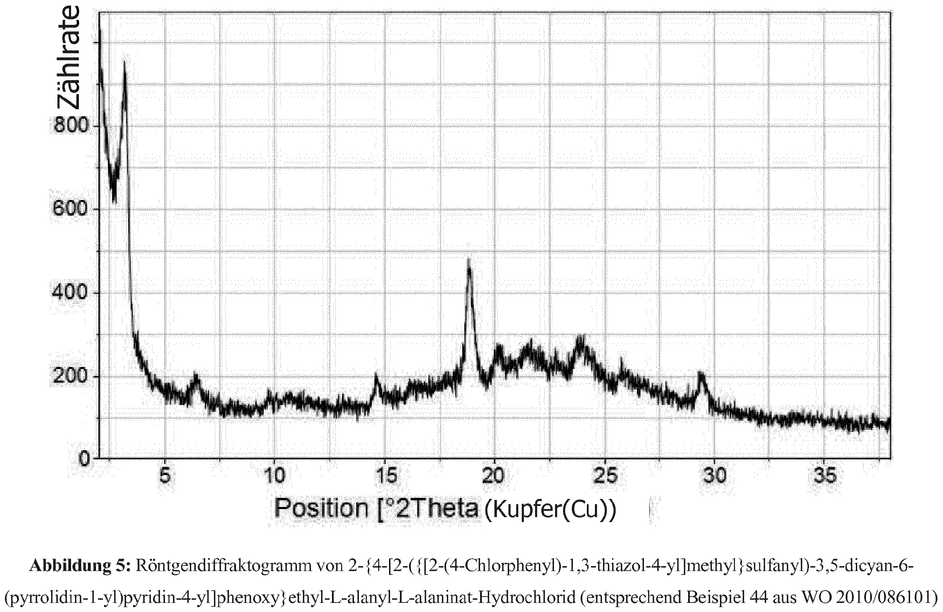

Das Röntgendiffraktogramm dieser Zielverbindung wurde unter den unten genannten Bedingungen erhalten (siehe Abbildung 5).

### HPLC-Bedingungen/Methoden

### Methode A

Zorbax Bonus RP; 150 × 3 mm; 3,5 µm
Ofentemperatur: 40 °C; Injektionsvolumen: 2 µL; Flussrate: 0,5 mL/min
Mobile Phase A: 1,36 g Kaliumdihydrogenphosphat + 1,15 g konz. Phosphorsäure (85% in Wasser) / 1 L Wasser;
Mobile Phase B: Acetonitril;
Linearer Gradient mit Stufen: 0': 10 % B; 12': 66 % B; 25': 80 % B; 35': 80 % B
UV-Detektion: 0' bis 4': 265 nm, 4' bis 35': 300 nm.

### Methode B

Zorbax Bonus RP; 150 × 3 mm; 3,5 µm
Ofentemperatur: 40 °C; Injektionsvolumen: 2 µL; Flussrate: 0,7 mL/min
Mobile Phase A: 1,36 g Kaliumdihydrogenphosphat + 1,15 g konz. Phosphorsäure (85% in Wasser) / 1 L Wasser;
Mobile Phase B: Acetonitril;
Linearer Gradient mit Stufen: 0': 50 % B; 20': 80 % B; 50': 80 % B
UV-Detektion: 300 nm.

### Methode C

Zorbax Bonus RP; 150 × 3 mm; 3,5 µm
Ofentemperatur: 40 °C; Injektionsvolumen: 3 µL; Flussrate: 0,7 mL/min
Mobile Phase A: 1,36 g Kaliumdihydrogenphosphat + 1,15 g konz. Phosphorsäure (85% in Wasser) + 1 g Hexansulfonsäure-Natrium Salz / 1 L Wasser;
Mobile Phase B: 50 mL Methanol / 1 L Acetonitril;
Linearer Gradient mit Stufen: 0': 10 % B; 5': 40 % B; 13': 50 % B; 20': 75 % B; 30': 80 % B; 35': 80 % B
UV-Detektion: 250 nm.

### Methode D

Zorbax RRHD Eclipse Plus C8; 100 × 2,1 mm; 1,8 µm
Ofentemperatur: 30 °C; Injektionsvolumen: 2 µL; Flussrate: 0,5 mL/min
Mobile Phase A: 0,5 mL konz. Phosphorsäure (85% in Wasser) / 1 L Wasser;
Mobile Phase B: 300 mL Methanol / 1 L Acetonitril;
Linearer Gradient mit Stufen: 0': 60 % B; 20': 80 % B; 30': 95 % B
UV-Detektion: 290 nm.

### Methode E

Zorbax YMC Triart C18; 100 × 3 mm; 1,9 µm
Ofentemperatur: 20 °C; Injektionsvolumen: 3 µL; Flussrate: 0,6 mL/min
Mobile Phase A: 1,5 g Ammoniumacetat + 3.5 mL 1%ige Essigsäure in Wasser / 1 L Wasser;
Mobile Phase B: 50 mL Methanol / 1 L Acetonitril;
Linearer Gradient mit Stufen: 0': 53 % B; 30': 70 % B; 40': 75 % B; 45': 75 % B
UV-Detektion: 300 nm.

### Messparamater der Röntgendiffraktometrie für die Messung der Verbindung der Formel (I) in kristalliner Form der Modifikationen I, II, III und IV:

| | |
|---|---|
| Scan-Achse | 2 Theta |
| Temperatur der Messung [°C] | 25 |
| Anodenmaterial | Cu |
| K-Alpha1 [Å] | 1,54060 |
| Generatoreinstellung | 40 mA, 40 kV |
| Diffraktometer Typ | Transmission diffractometer |
| Primärstrahl-Monochromator | Ja |
| Probendrehung | Ja |

**Tabelle 1: Peak Maxima des 2 Theta Winkels**

| Peak Maximum [2 Theta] | | | |
|---|---|---|---|
| Modifikation I | Modifikation II | Modifikation III | Modifikation IV |
| 4.3 | 3.7 | 3.1 | 3.0 |
| 5.4 | 4.0 | 6.2 | 6.0 |
| 6.5 | 6.4 | 8.0 | 9.0 |
| 8.7 | 7.5 | 10.0 | 12.1 |
| 9.8 | 9.1 | 10.4 | 13.7 |
| 10.8 | 9.5 | 12.3 | 15.3 |
| 11.8 | 10.5 | 13.2 | 17.0 |
| 13.0 | 12.5 | 15.2 | 20.8 |
| 13.9 | 13.7 | 15.7 | 21.3 |
| 14.9 | 14.1 | 16.5 | 22.8 |
| 15.4 | 14.8 | 18.3 | 25.2 |
| 16.0 | 15.1 | 19.2 | 27.5 |
| 16.3 | 15.7 | 19.8 | 29.2 |
| 17.1 | 16.3 | 21.1 | 31.0 |
| 18.2 | 17.0 | 22.0 | |
| 18.3 | 17.5 | 22.7 | |
| 18.9 | 19.3 | 24.0 | |
| 19.7 | 19.7 | 26.0 | |
| 19.9 | 22.8 | 27.0 | |
| 20.2 | 23.8 | 29.7 | |
| 20.7 | 24.9 | 31.5 | |
| 21.5 | 25.4 | 32.5 | |
| 22.6 | 26.7 | 33.7 | |
| 23.0 | 27.5 | | |
| 23.5 | 34.7 | | |
| 23.7 | | | |
| 24.3 | | | |
| 24.9 | | | |
| 25.6 | | | |
| 26.3 | | | |
| 27.4 | | | |
| 28.4 | | | |
| 29.3 | | | |
| 30.3 | | | |
| 31.6 | | | |
| 32.9 | | | |
| 35.8 | | | |
| 37.3 | | | |

### Messbedingungen für die Raman-Spektroskopie für die Messung der Verbindung der Formel (I) in kristalliner Form der Modifikationen I, II, III und IV sowie in Form des Isopropanol-Solvates:

| | |
|---|---|
| Gerät | Bruker Raman RFS 100/S |
| Anzahl Scans | 64 |
| Auflösung | 2 - 4 cm⁻¹ |
| Laser Power | 50 mW |
| Laser Wellenlänge | 1064 nm |

**Tabelle 2: Bandenmaxima [cm⁻¹] im Raman-Spektrum**

| Bandenmaxima [cm⁻¹] | | | | |
|---|---|---|---|---|
| Modifikation I | Modifikation II | Modifikation III | Modifikation IV | Isopropanol-Solvat |
| 3102 | 3066 | 3071 | 3073 | 3090 |
| 3069 | 2944 | 2973 | 2988 | 3074 |
| 2965 | 2212 | 2946 | 2942 | 2987 |
| 2939 | 1607 | 2879 | 2883 | 2939 |
| 2907 | 1595 | 2217 | 2212 | 2880 |
| 2211 | 1534 | 1609 | 1610 | 2209 |
| 1610 | 1501 | 1596 | 1597 | 1728 |
| 1596 | 1454 | 1546 | 1523 | 1666 |
| 1539 | 1401 | 1525 | 1503 | 1609 |
| 1515 | 1323 | 1497 | 1451 | 1595 |
| 1498 | 1241 | 1449 | 1400 | 1551 |
| 1447 | 1188 | 1400 | 1326 | 1539 |
| 1402 | 1135 | 1326 | 1224 | 1526 |
| 1394 | 1093 | 1294 | 1186 | 1517 |
| 1338 | 739 | 1267 | 1112 | 1495 |
| 1325 | 650 | 1185 | 1093 | 1447 |
| 1295 | 526 | 1162 | 737 | 1399 |
| 1245 | 419 | 1133 | 650 | 1391 |
| 1225 | | 1090 | 629 | 1339 |
| 1182 | | 774 | 529 | 1320 |
| 1163 | | 737 | 444 | 1295 |
| 1133 | | 647 | 417 | 1253 |
| 1095 | | 627 | 361 | 1231 |
| 1018 | | 531 | 322 | 1174 |
| 1004 | | 506 | | 1136 |
| 883 | | 424 | | 1092 |
| 783 | | 362 | | 1015 |
| 736 | | 317 | | 956 |
| 646 | | 272 | | 939 |
| 627 | | | | 921 |
| 598 | | | | 879 |
| 527 | | | | 819 |
| 506 | | | | 781 |
| 420 | | | | 763 |
| 361 | | | | 734 |
| 337 | | | | 645 |
| 315 | | | | 627 |
| 294 | | | | 529 |
| 270 | | | | 505 |
| 227 | | | | 453 |
| | | | | 421 |
| | | | | 361 |
| | | | | 328 |
| | | | | 315 |
| | | | | 267 |
| | | | | 225 |
| | | | | 120 |
| | | | | 163 |

### Beschreibung der Abbildungen:

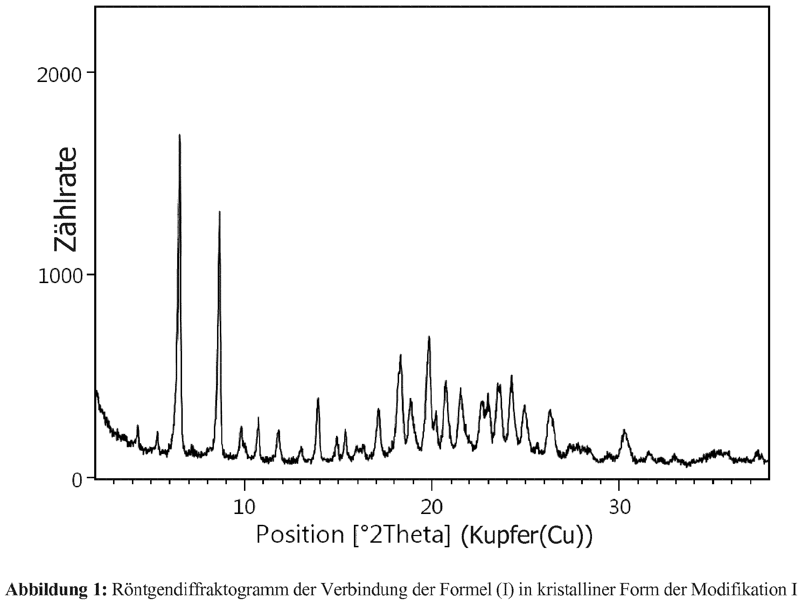

**Abbildung 1****:** Röntgendiffraktogramm der Verbindung der Formel (I) in kristalliner Form der Modifikation I

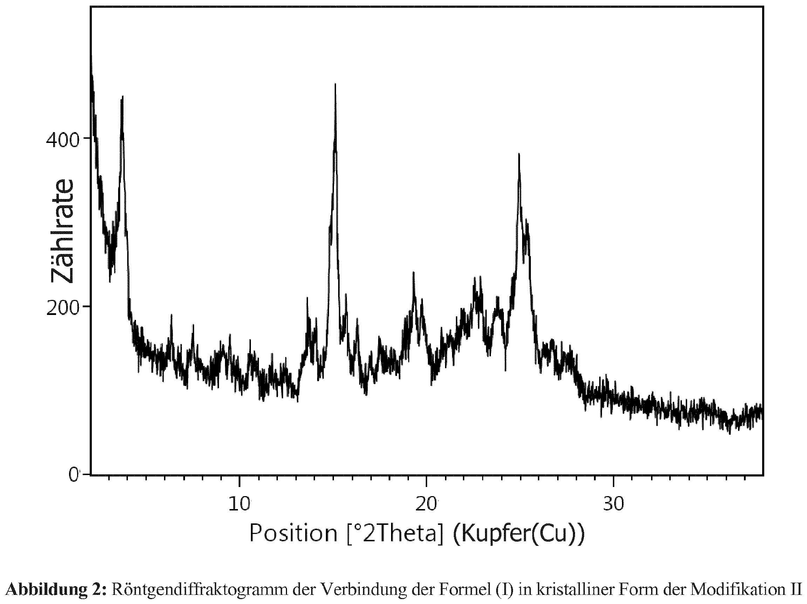

**Abbildung 2****:** Röntgendiffraktogramm der Verbindung der Formel (I) in kristalliner Form der Modifikation II

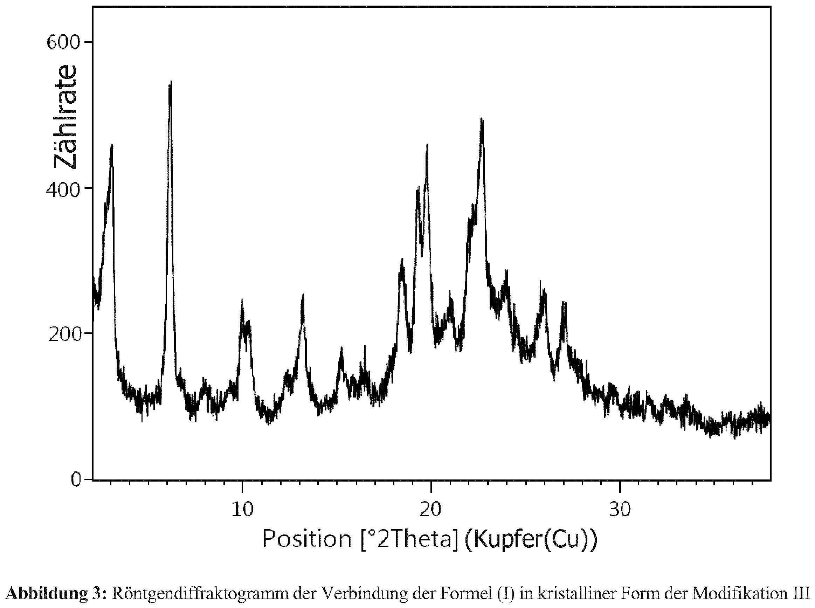

**Abbildung 3****:** Röntgendiffraktogramm der Verbindung der Formel (I) in kristalliner Form der Modifikation III

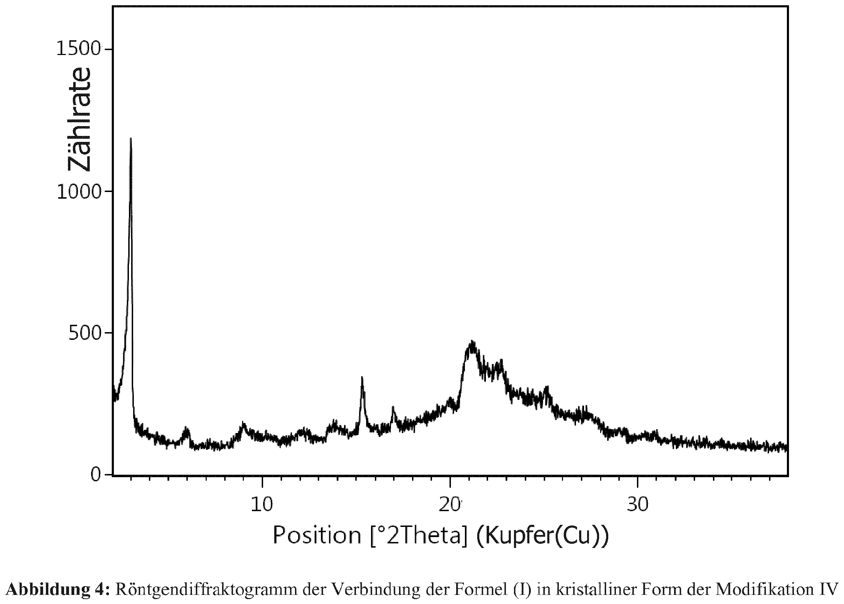

**Abbildung 4****:** Röntgendiffraktogramm der Verbindung der Formel (I) in kristalliner Form der Modifikation IV
**Abbildung 5****:** Röntgendiffraktogramm der Verbindung der Formel (I) in kristalliner Form der Modifikation IV

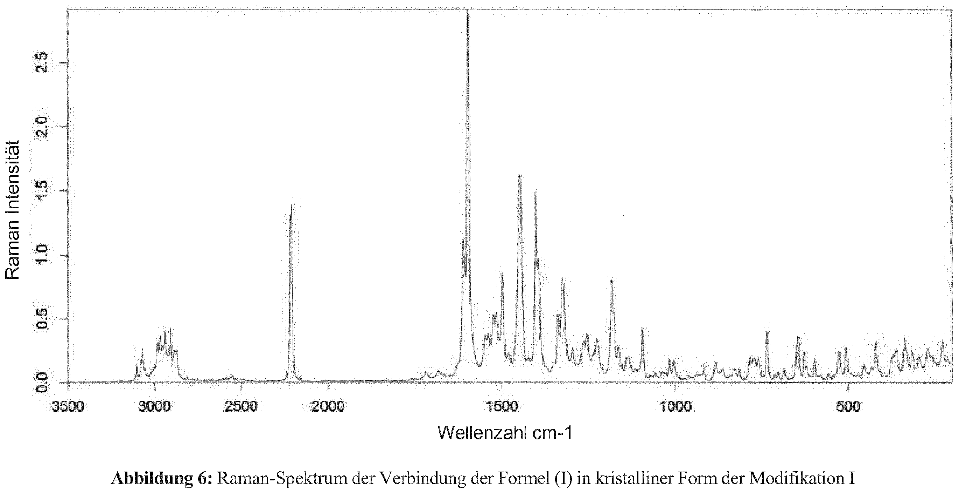

**Abbildung 6****:** Raman-Spektrum der Verbindung der Formel (I) in kristalliner Form der Modifikation I

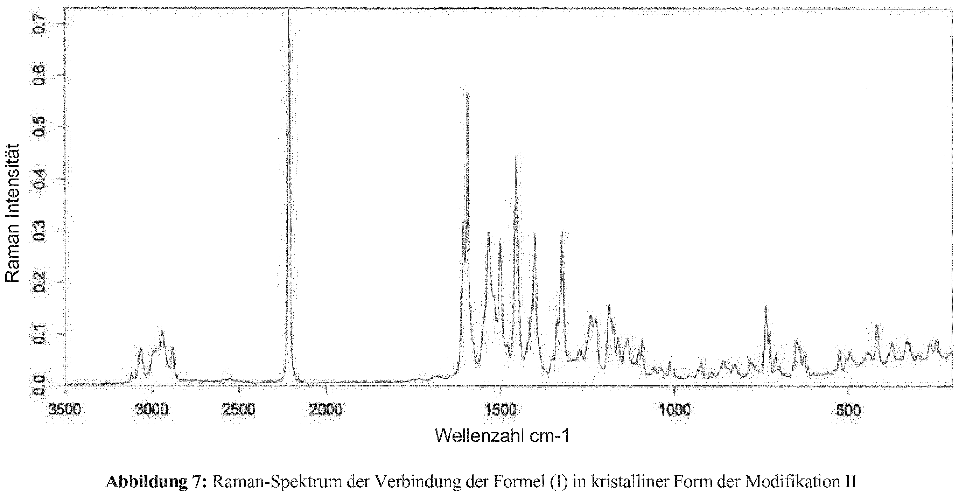

**Abbildung 7****:** Raman-Spektrum der Verbindung der Formel (I) in kristalliner Form der Modifikation II

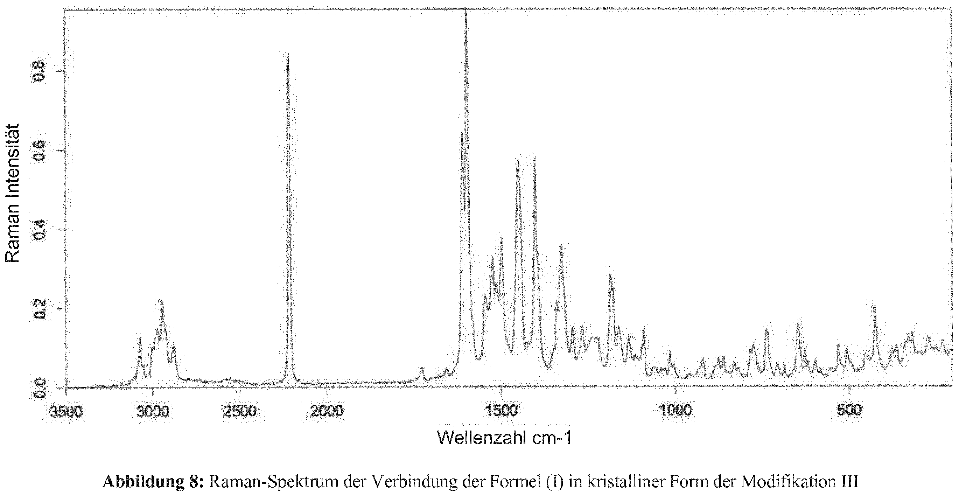

**Abbildung 8****:** Raman-Spektrum der Verbindung der Formel (I) in kristalliner Form der Modifikation III

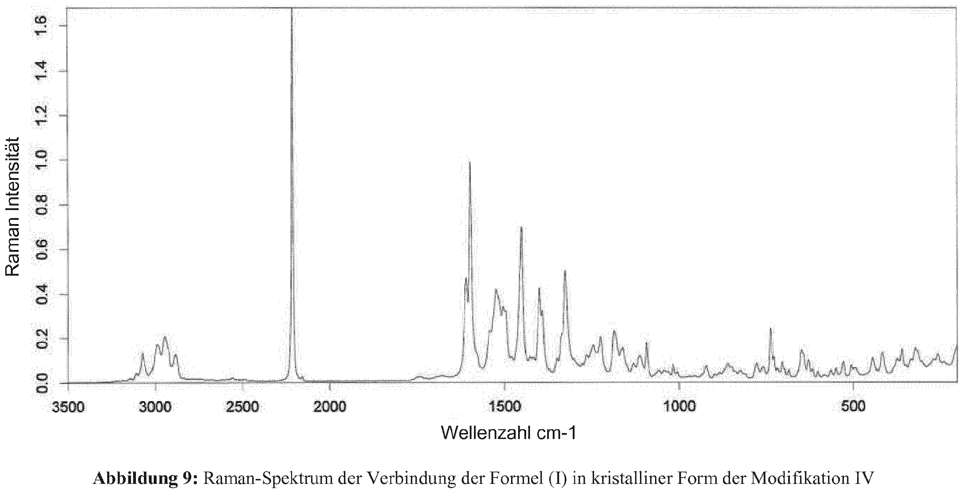

**Abbildung 9****:** Raman-Spektrum der Verbindung der Formel (I) in kristalliner Form der Modifikation IV

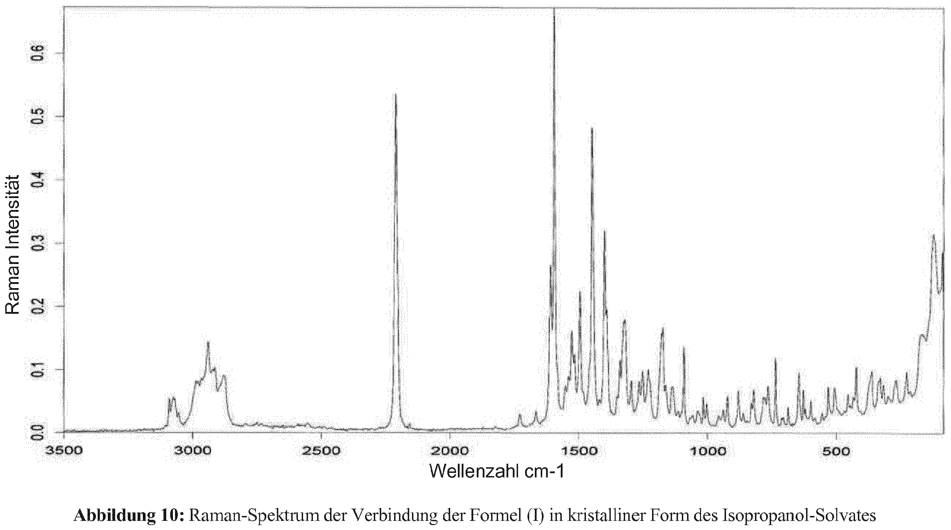

**Abbildung 10****:** Raman-Spektrum der Verbindung der Formel (I) in kristalliner Form des Isopropanol-Solvates

## Patentansprüche

1. 2-{4-[2-({[2-(4-Chlorphenyl)-1,3-thiazol-4-yl]methyl}sulfanyl)-3,5-dicyan-6-(pyrrolidin-1-yl)-pyridin-4-yl]phenoxy}ethyl-L-alanyl-L-alaninat-Monohydrochlorid der Formel (I) in kristalliner Form der Modifikation I 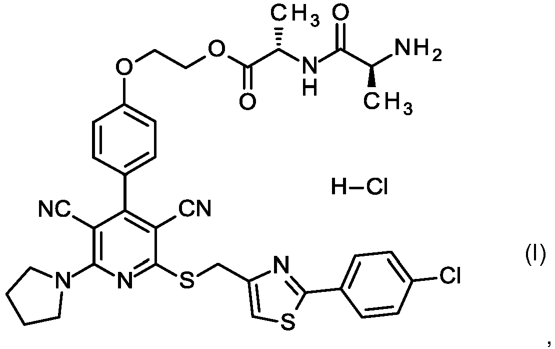 **dadurch gekennzeichnet, dass** das Röntgendiffraktogramm der Verbindung Peakmaxima des 2 Theta Winkels bei 6.5, 8.7 und 24.3 zeigt, gemessen unter Verwendung von Kupfer-K-Alpha1-Strahlung (1.54060Å).

2. Verbindung nach Anspruch 1, **dadurch gekennzeichnet, dass** das Röntgendiffraktogramm der Verbindung Peakmaxima des 2 Theta Winkels bei 6.5, 8.7, 18.3, 19.9, 20.7, 23.5 und 24.3 zeigt, gemessen unter Verwendung von Kupfer-K-Alpha1-Strahlung (1.54060Å).

3. 2-{4-[2-({[2-(4-Chlorphenyl)-1,3-thiazol-4-yl]methyl}sulfanyl)-3,5-dicyan-6-(pyrrolidin-1-yl)-pyridin-4-yl]phenoxy}ethyl-L-alanyl-L-alaninat-Monohydrochlorid der Formel (I) in kristalliner Form der Modifikation I 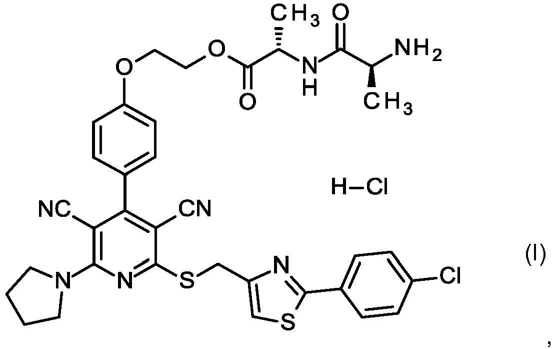 **dadurch gekennzeichnet, dass** das Raman-Spektrum der Verbindung Bandenmaxima bei 2907, 1004 und 598 cm⁻¹ zeigt.

4. Verbindung nach Anspruch 3, **dadurch gekennzeichnet, dass** das Raman-Spektrum der Verbindung Bandenmaxima bei 2907, 1539, 1515, 1182, 1004 und 598 cm⁻¹ zeigt.

5. Verbindung nach Anspruch 3 oder 4, **dadurch gekennzeichnet, dass** das Raman-Spektrum der Verbindung Bandenmaxima bei 2907, 1539, 1515, 1394, 1245, 1182, 1004 und 598 cm⁻¹ zeigt.

6. Arzneimittel enthaltend eine Verbindung gemäß einem der Ansprüche 1 bis 5 und keine größeren Anteile einer anderen Form der Verbindung der Formel (I).

7. Arzneimittel enthaltend eine Verbindung gemäß einem der Ansprüche 1 bis 5 in mehr als 90 Gewichtsprozente bezogen auf die Gesamtmenge der enthaltenen Verbindung der Formel (I).

8. Verbindung gemäß einem der Ansprüche 1 bis 5 zur Anwendung in einem Verfahren für die Behandlung von Herz-Kreislauferkrankungen oder von Nierenerkrankungen.

9. Verfahren zur Herstellung von 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-alanyl-L-alaninat-Monohydrochlorid der Formel (I) in kristalliner Form der Modifikation I, welche die den Ansprüchen 1 bis 5 angegebenen Peakmaxima im Röntgendiffraktogramm bzw. Bandenmaxima im Raman-Spektrum aufweist, **dadurch gekennzeichnet, dass** das Dialaninat-Dihydrochlorid der Formel (IX) 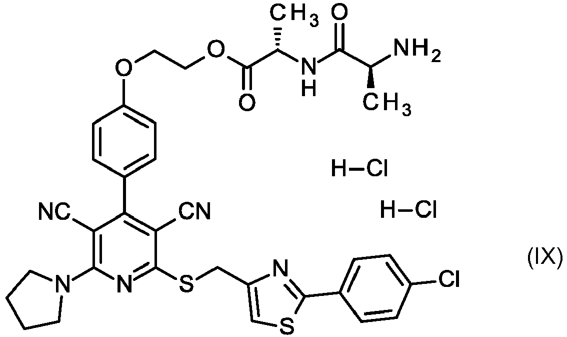 in einem Überschuss an Isopropanol oder n-Propanol/Wasser-Gemisch (98:2) bei Raumtemperatur rührt und die Verbindung der Formel (I) in der kristallinen Modifikation I nach dem Trocknen erhalten wird.

10. Verfahren gemäß Anspruch 9 zur Herstellung von Verbindung (I) 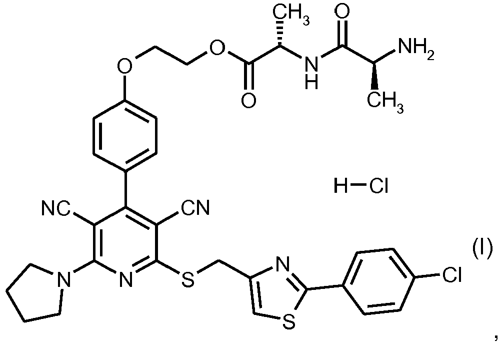 **dadurch gekennzeichnet, dass**
[A] in Stufe 1 in einer Eintopfreaktion die Verbindung der Formel (II) 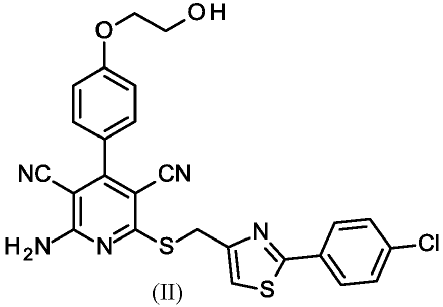 in Gegenwart eines Lösungsmittels, eines Phasentransferkatalysator, eines Nitrits und Kupfer(II)chlorids umsetzt und das erhaltene Zwischenprodukt der Formel (III) 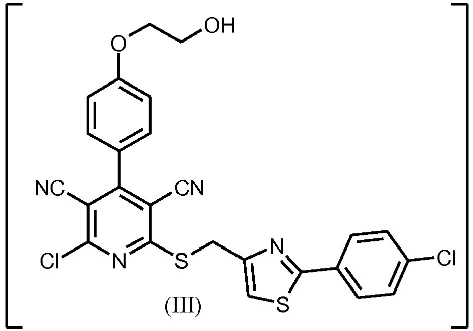 ohne Isolierung, d.h. in Lösung, mit Pyrrolidin zur Verbindung der Formel (IV) 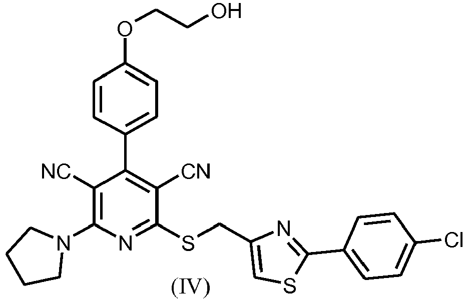 umsetzt, und
[B] diese so erhaltene Verbindung der Formel (IV) in einer Stufe 2 in einer Eintopfreaktion mit Boc-L-Alanin in Gegenwart des Kondensationsmittels Dicyclohexylcarbodiimid (DCC) in Kombination mit 4-(Dimethylamino)-pyridin und in Gegenwart eines Lösungsmittels zum geschützten Boc-Alaninat (V) 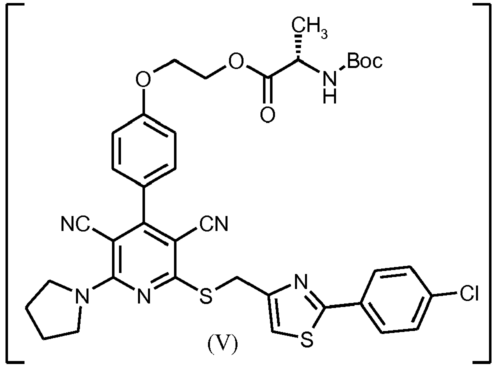 umsetzt, welches ohne Isolierung, d.h. in Lösung, mit Salzsäure zum Alaninat-Dihydrochlorid der Formel (VIII) 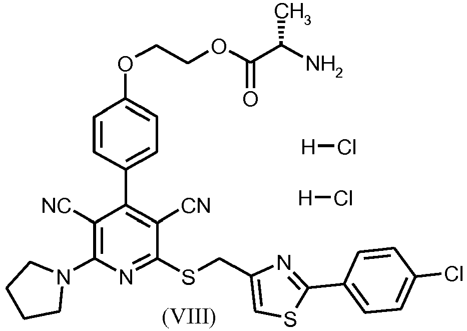 umgesetzt wird, und
[C] diese so erhaltene Verbindung der Formel (VIII) in einer Stufe 3 in Kombination mit 4-(Dimethylamino)-pyridin und in Gegenwart eines Lösungsmittels mit Boc-L-Alanin in Gegenwart des Kondensationsmittels Dicyclohexylcarbodiimid (DCC) zum geschützten Boc-Dialaninat (VII) 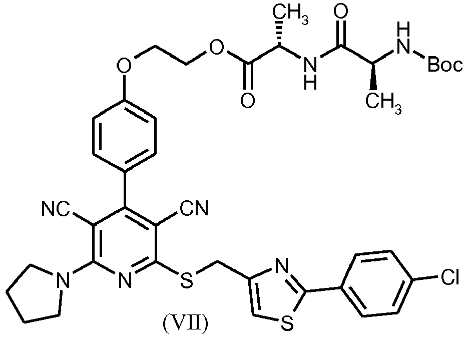 umsetzt, und
[D] diese so erhaltene Verbindung der Formel (VII) in einer Stufe 4 in Gegenwart eines Lösungsmittels mit Salzsäure zum Dialaninat-Dihydrochlorid der Formel (IX) 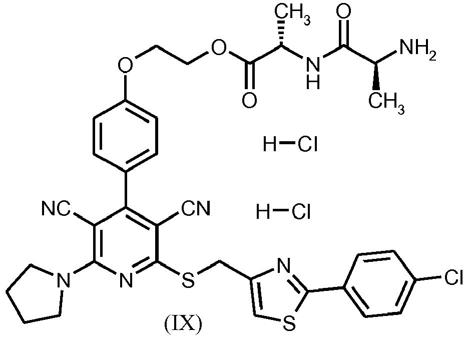 umgesetzt, und
[E] diese so erhaltene Verbindung der Formel (IX) in einer Stufe 5 in einem Überschuss an Isopropanol oder n-Propanol/Wasser-Gemisch (98:2) bei Raumtemperatur rührt und die Verbindung der Formel (I) in der kristallinen Modifikation I isoliert.

11. Verfahren nach Anspruch 10, **dadurch gekennzeichnet, dass** man die Verbindung der Formel (II) 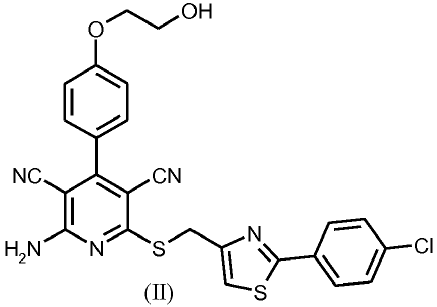 erhält, indem man in Gegenwart von Methanol und Triethylamin zunächst das substituierte Malononitril der Formel (XI) 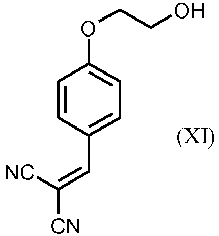 mit Cyanthioacetamid der Formel (XII) 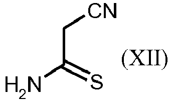 umsetzt und das erhaltene Zwischenprodukt anschließen mit Chlormethylchlorphenylthiazol der Formel (XIV) 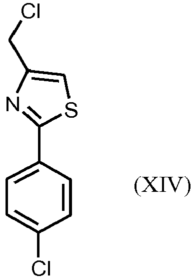 umsetzt.

12. Verfahren nach Anspruch 10, **dadurch gekennzeichnet, dass** man in Stufe 1 das Zwischenprodukt der Formel (III) isoliert, das heißt als Feststoff erhält, bevor es weiter umgesetzt wird.

13. Verfahren nach Anspruch 10, **dadurch gekennzeichnet, dass** man in Stufe 2 das geschützte Boc-Alaninat der Formel (V) isoliert, das heißt als Feststoff erhält, bevor es weiter umgesetzt wird.

14. Verfahren nach Anspruch 10, **dadurch gekennzeichnet, dass** man das in Stufe 3 erhaltene geschützte Boc-Dialaninat der Formel (VII) nicht isoliert, sondern direkt weiter umsetzt.

15. Verfahren nach Anspruch 10, **dadurch gekennzeichnet, dass** man in Stufe 1 das Zwischenprodukt der Formel (III) isoliert, das heißt als Feststoff erhält, bevor es weiter umgesetzt wird

16. Verbindung 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-alanyl-L-alaninat-Dihydrochlorid der Formel (IX)

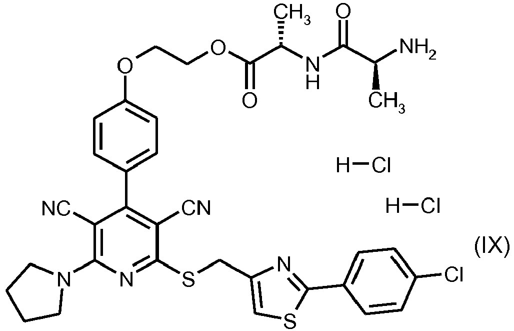

17. Verbindung 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-alaninat-Dihydrochlorid (VIII)

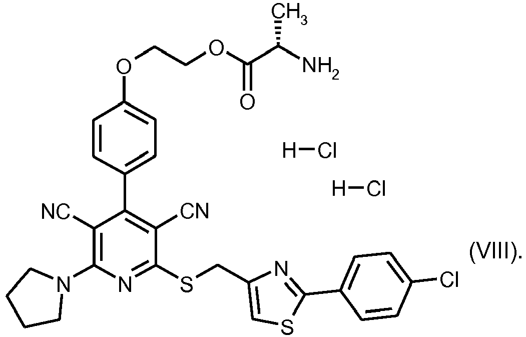

## Claims

1. 2-(4-[2-(1[2-(4-Chlorophenyl)-1,3-thiazol-4-yl]methyl}sulphanyl)-3,5-dicyano-6-(pyrrolidin-1-yl)pyridin-4-yl]phenoxy}ethyl-L-alanyl L-alaninate monohydrochloride of the formula (I) in crystalline form of modification I 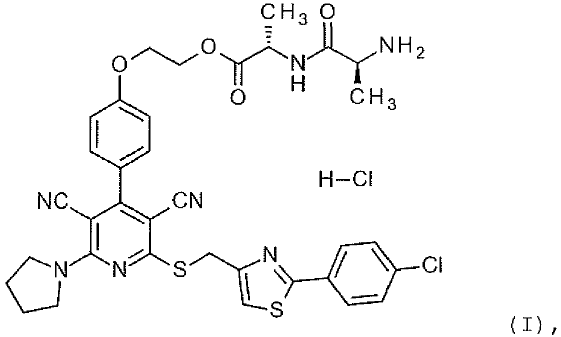 **characterized in that** the X-ray diffractogram of the compound has peak maxima of the 2 theta angle at 6.5, 8.7 and 24.3, measured using copper K alpha1 radiation (1.54060 Å).

2. Compound according to Claim 1, **characterized in that** the X-ray diffractogram of the compound has peak maxima of the 2 theta angle at 6.5, 8.7, 18.3, 19.9, 20.7, 23.5 and 24.3, measured using copper K alpha1 radiation (1.54060 Å).

3. 2-{4-[2-({[2-(4-Chlorophenyl)-1,3-thiazol-4-yl]methyl}sulphanyl)-3,5-dicyano-6-(pyrrolidin-1-yl)pyridin-4-yl]phenoxy}ethyl-L-alanyl L-alaninate monohydrochloride of the formula (I) in crystalline form of modification I 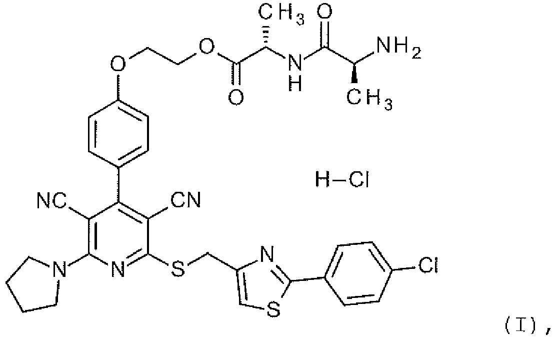 **characterized in that** the Raman spectrum of the compound has band maxima at 2907, 1004 and 598 cm⁻¹.

4. Compound according to Claim 3, **characterized in that** the Raman spectrum of the compound has band maxima at 2907, 1539, 1515, 1182, 1004 and 598 cm⁻¹.

5. Compound according to Claim 3 or 4, **characterized in that** the Raman spectrum of the compound has band maxima at 2907, 1539, 1515, 1394, 1245, 1182, 1004 and 598 cm⁻¹.

6. Medicament comprising a compound according to any of Claims 1 to 5 and no greater proportions of any other form of the compound of the formula (I).

7. Medicament comprising a compound according to any of Claims 1 to 5 in more than 90 per cent by weight based on the total amount of the compound of the formula (I) present.

8. Compound according to any of Claims 1 to 5 for use in a method for treatment of cardiovascular disorders or of kidney disorders.

9. Process for preparing 2-{4-[2-({[2-(4-chlorophenyl)-1,3-thiazol-4-yl]methyl}sulphanyl)-3,5-dicyano-6-(pyrrolidin-1-yl)pyridin-4-yl]phenoxy}ethyl-L-alanyl L-alaninate monohydrochloride of the formula (I) in crystalline form of modification I, which has the X-ray diffractogram peak maxima and Raman spectrum band maxima stated in Claims 1 to 5, **characterized in that** the dialaninate dihydrochloride of the formula (IX) 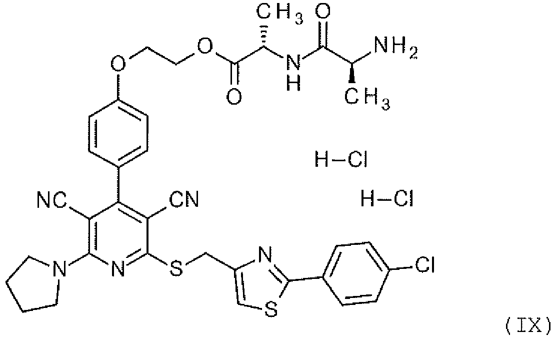 is stirred in an excess of isopropanol or n-propanol/water mixture (98:2) at room temperature, giving, after drying, the compound of the formula (I) in crystalline modification I.

10. Process according to Claim 9 for preparing compound (I) 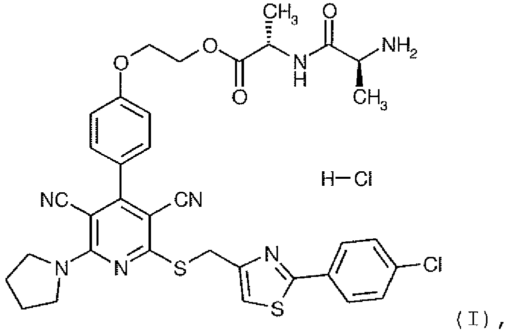 **characterized in that**
[A] in step 1 in a one-pot reaction the compound of the formula (II) 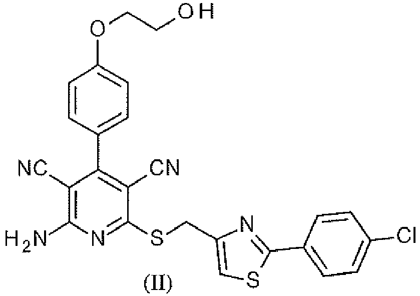 is reacted in the presence of a solvent, a phase transfer catalyst, a nitrite and copper(II) chloride and the intermediate of the formula (III) obtained 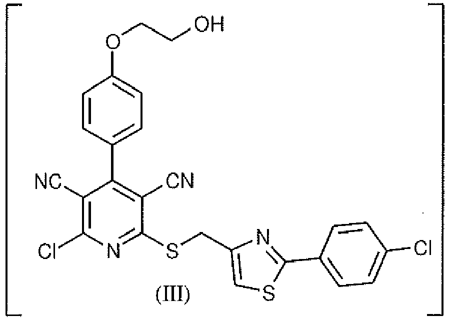 is reacted without isolation, i.e. in solution, with pyrrolidine to give the compound of the formula (IV) 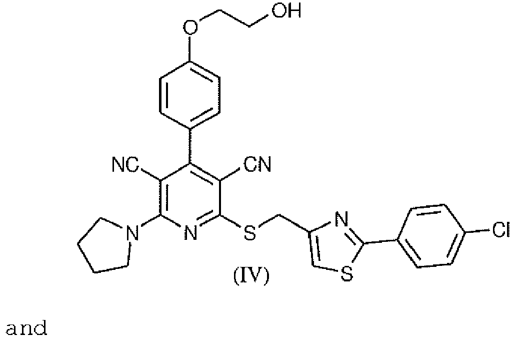
[B] the compound of the formula (IV) obtained in this manner is, in a step 2, converted in a one-pot reaction with Boc-L-alanine in the presence of the condensing agent dicyclohexylcarbodiimide (DCC) in combination with 4-(dimethylamino)pyridine and in the presence of a solvent into the protected Boc-alaninate (V) 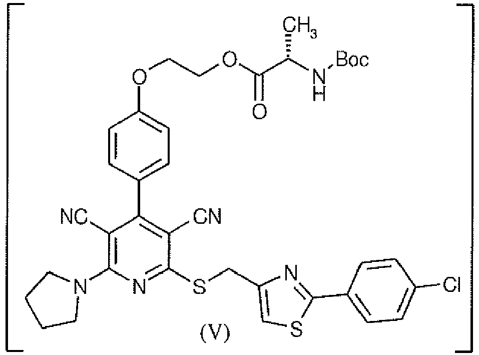 which is, without isolation, i.e. in solution, converted with hydrochloric acid into the alaninate dihydrochloride of the formula (VIII) 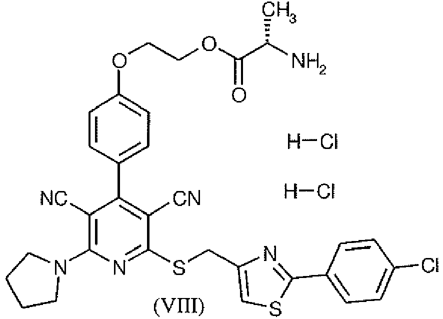 and
[C] the compound of the formula (VIII) obtained in this manner is, in a step 3, in combination with 4-(dimethylamino)pyridine and in the presence of a solvent, reacted with Boc-L-alanine in the presence of the condensing agent dicyclohexylcarbodiimide (DCC) to give the protected Boc-dialaninate (VII) 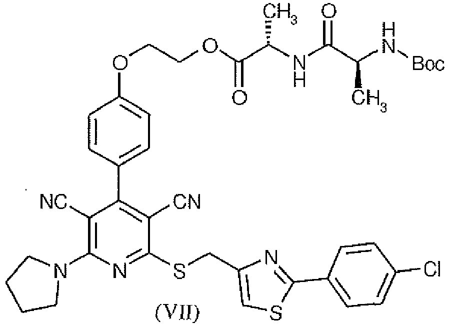 and
[D] the compound of the formula (VII) obtained in this manner is, in a step 4, in the presence of a solvent, reacted with hydrochloric acid to give the dialaninate dihydrochloride of the formula (IX) 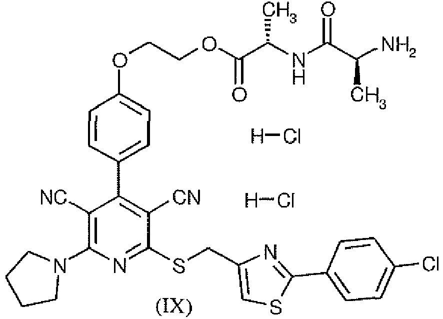 and
[E] the compound of the formula (IX) obtained in this manner is, in a step 5, stirred in an excess of isopropanol or n-propanol/water mixture (98:2) at room temperature, giving the compound of the formula (I) in crystalline modification I.

11. Process according to Claim 10, **characterized in that** the compound of the formula (II) 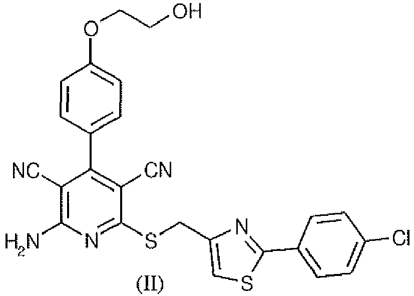 is obtained by first, in the presence of methanol and triethylamine, reacting the substituted malononitrile of the formula (XI) 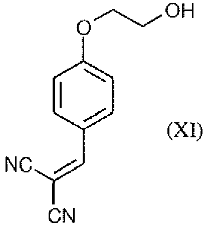 with cyanothioacetamide of the formula (XII) 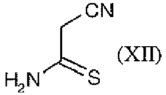 and then reacting the intermediate obtained with chloromethylchlorephenylthiazole of the formula (XIV)

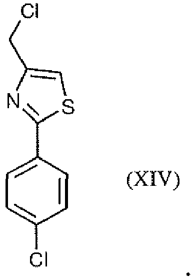

12. Process according to Claim 10, **characterized in that** in step 1 the intermediate of the formula (III) is isolated, that is obtained as a solid, prior to being reacted further.

13. Process according to Claim 10, **characterized in that** in step 2 the protected Boc-alaninate of the formula (V) is isolated, that is obtained as a solid, prior to being reacted further.

14. Process according to Claim 10, **characterized in that** the protected Boc-dialaninate of the formula (VII) obtained in step 3 is not isolated, but directly reacted further.

15. Process according to Claim 10, **characterized in that** in step 1 the intermediate of the formula (III) is isolated, that is obtained as a solid, prior to being reacted further.

16. Compound 2-{4-[2-({[2-(4-chlorophenyl)-1,3-thiazol-4-yl]methyl}sulphanyl)-3,5-dicyano-6-(pyrrolidin-1-yl)pyridin-4-yl]phenoxy}ethyl-L-alanyl L-alaninate dihydrochloride of the formula (IX)

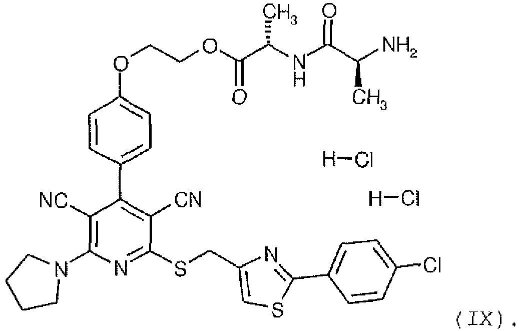

17. Compound 2-{4-[2-({[2-(4-chlorophenyl)-1,3-thiazol-4-yl]methyl}sulphanyl)-3,5-dicyano-6-(pyrrolidin-1-yl)pyridin-4-yl]phenoxy}ethyl L-alaninate dihydrochloride (VIII)

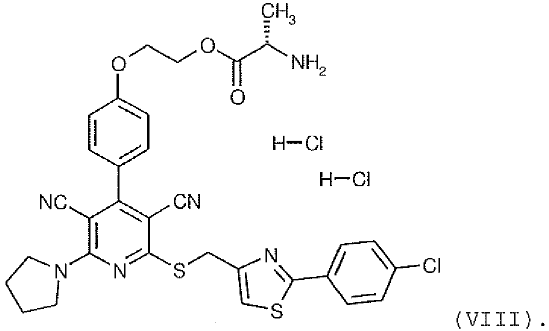

## Revendications

1. Monochlorhydrate de L-alaninate de 2-{4-[2-({[2-(4-chlorophényl)-1,3-thiazol-4-yl]méthyl}sulfanyl)-3,5-dicyano-6-(pyrrolidin-1-yl)-pyridin-4-yl]phénoxy}éthyl-L-alanyle de formule (I) sous la forme cristalline I : 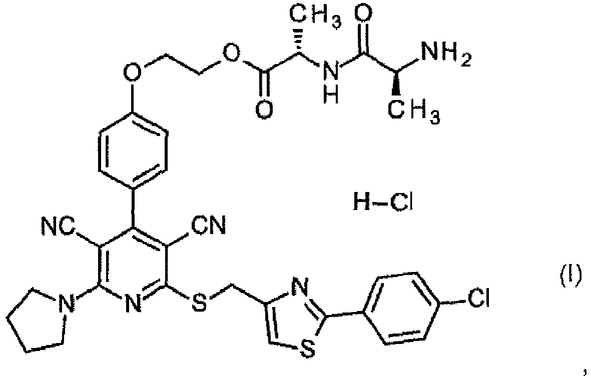 **caractérisé en ce que** le diffractogramme de rayons X du composé présente des maximums de pic de l'angle 2-thêta à 6,5, 8,7 et 24,3, mesurés en utilisant un rayonnement cuivre-K-alpha 1 (1,54060 Å).

2. Composé selon la revendication 1, **caractérisé en ce que** le diffractogramme de rayons X du composé présente des maximums de pic de l'angle 2-thêta à 6,5, 8,7, 18,3, 19,9, 20,7, 23,5 et 24,3, mesurés en utilisant un rayonnement cuivre-K-alpha 1 (1,54060 Å).

3. Monochlorhydrate de L-alaninate de 2-{4-[2-({[2-(4-chlorophényl)-1,3-thiazol-4-yl]méthyl}sulfanyl)-3,5-dicyano-6-(pyrrolidin-1-yl)-pyridin-4-yl]phénoxy}éthyl-L-alanyle de formule (I) sous la forme cristalline I : 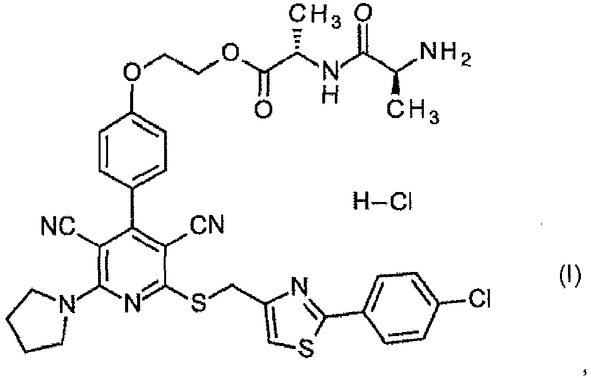 **caractérisé en ce que** le spectre de Raman du composé présente des maximums de bande à 2 907, 1 004 et 598 cm⁻¹

4. Composé selon la revendication 3, **caractérisé en ce que** le spectre de Raman du composé présente des maximums de bande à 2 907, 1 539, 1 515, 1 182, 1 004 et 598 cm⁻¹.

5. Composé selon la revendication 3 ou 4, **caractérisé en ce que** le spectre de Raman du composé présente des maximums de bande à 2 907, 1 539, 1 515, 1 394, 1 245, 1 182, 1 004 et 598 cm⁻¹.

6. Médicament contenant un composé selon l'une quelconque des revendications 1 à 5 et ne contenant pas de proportions plus importantes d'une autre forme du composé de formule (I).

7. Médicament contenant un composé selon l'une quelconque des revendications 1 à 5 à hauteur de plus de 90 pour cent en poids, par rapport à la quantité totale du composé de formule (I) contenu.

8. Composé selon l'une quelconque des revendications 1 à 5, destiné à une utilisation dans un procédé de traitement de maladies cardiovasculaires ou de maladies rénales.

9. Procédé de fabrication de monochlorhydrate de L-alaninate de 2-{4-[2-({[2-(4-chlorophényl)-1,3-thiazol-4-yl]méthyl}sulfanyl)-3,5-dicyano-6-(pyrrolidin-1-yl)-pyridin-4-yl]phénoxy}éthyl-L-alanyle de formule (I) sous la forme cristalline I, qui présente les maximums de pic sur le diffractogramme de rayons X et les maximums de bande sur le spectre de Raman indiqués dans les revendications 1 à 5, **caractérisé en ce que** le dichlorhydrate de dialaninate de formule (IX) 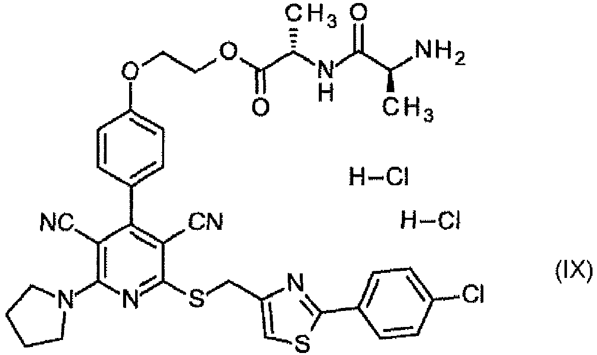 est agité dans un excès d'isopropanol ou un mélange n-propanol/eau (98:2) à température ambiante, et le composé de formule (I) sous la forme cristalline I est obtenu après le séchage.

10. Procédé selon la revendication 9 pour la fabrication du composé (I) : 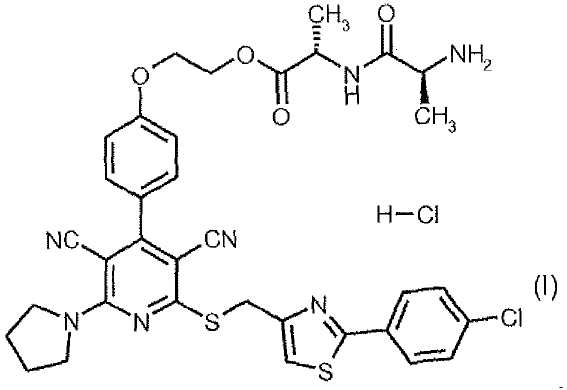 **caractérisé en ce que**
[A] à l'étape 1, par une réaction monotope, le composé de formule (II) 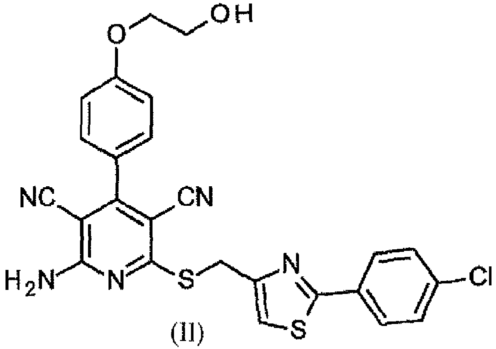 est mis en réaction en présence d'un solvant, d'un catalyseur de transfert de phase, d'un nitrite et de chlorure de cuivre (II), et le produit intermédiaire de formule (III) obtenu 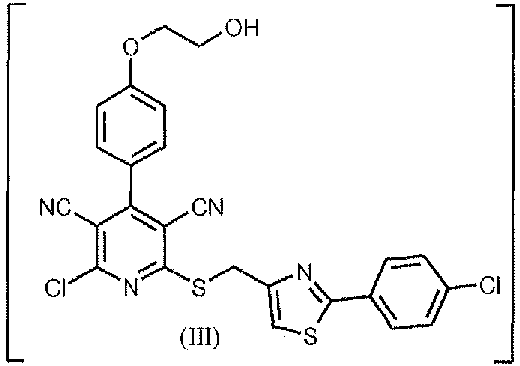 est mis en réaction sans isolement, c.-à-d. en solution, avec de la pyrrolidine pour former le composé de formule (IV) 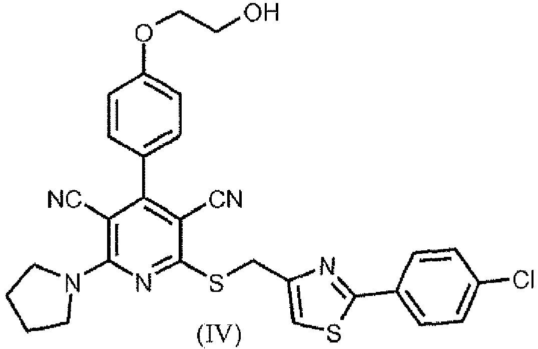 et
[B] le composé de formule (IV) ainsi obtenu est mis en réaction lors d'une étape 2 par une réaction monotope avec de la Boc-L-alanine en présence de l'agent de condensation dicyclohexylcarbodiimide (DCC) en combinaison avec de la 4-(diméthylamino)-pyridine et en présence d'un solvant pour former le Boc-alaninate protégé (V) 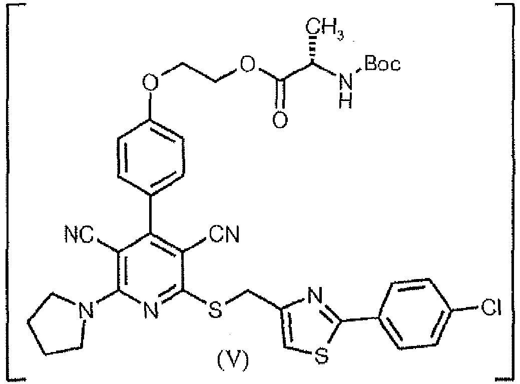 qui est mis en réaction sans isolement, c.-à-d. en solution, avec de l'acide chlorhydrique pour former le dichlorhydrate d'alaninate de formule (VIII) 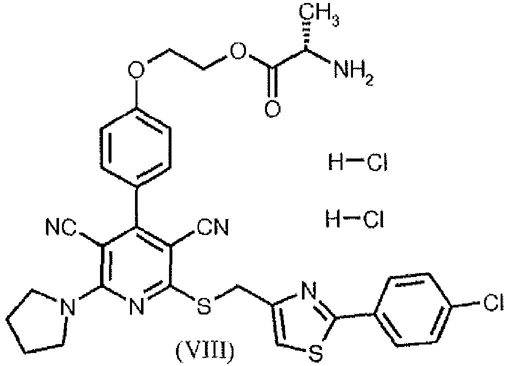 et
[C] ce composé de formule (VIII) ainsi obtenu est mis en réaction lors d'une étape 3 en combinaison avec de la 4-(diméthylamino)-pyridine et en présence d'un solvant avec de la Boc-L-alanine en présence de l'agent de condensation dicyclohexylcarbodiimide (DCC) pour former le Boc-dialaninate protégé (VII) 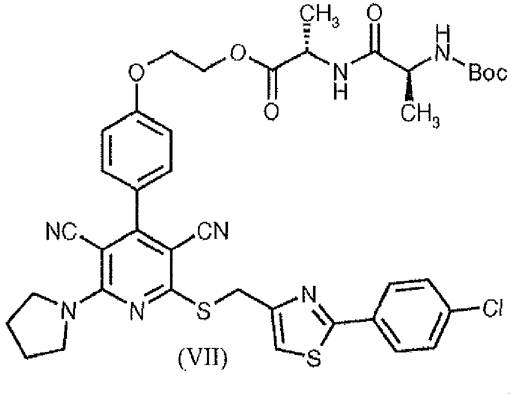 et
[D] ce composé de formule (VII) ainsi obtenu est mis en réaction lors d'une étape 4 en présence d'un solvant avec de l'acide chlorhydrique pour former le dichlorhydrate de dialaninate de formule (IX) 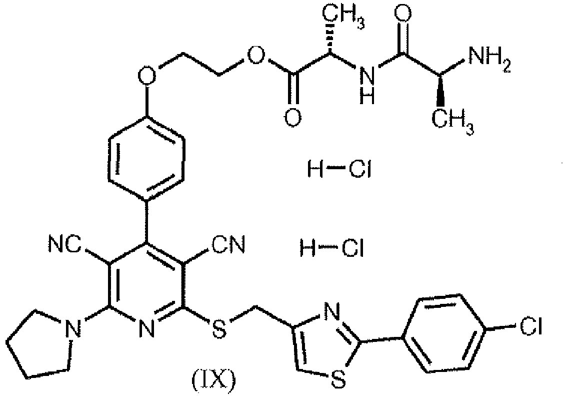 et
[E] ce composé de formule (IX) ainsi obtenu est agité lors d'une étape 5 dans un excès d'isopropanol ou un mélange n-propanol/eau (98:2) à température ambiante, et le composé de formule (I) sous la forme cristalline I est isolé.

11. Procédé selon la revendication 10, **caractérisé en ce que** le composé de formule (II) 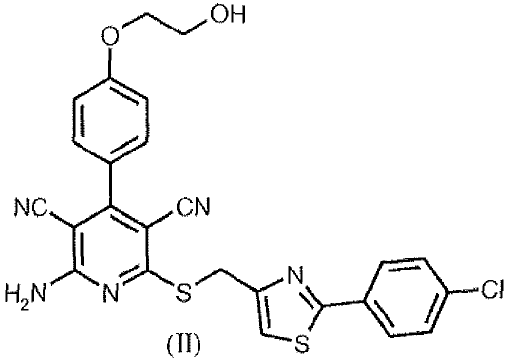 est obtenu tout d'abord par mise en réaction du malononitrile substitué de formule (XI) en présence de méthanol et de triéthylamine 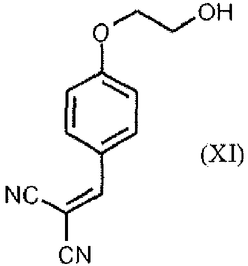 avec du cyanthioacétamide de formule (XII) 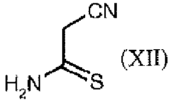 puis mise en réaction du produit intermédiaire obtenu avec du chlorométhylchlorophénylthiazole de formule (XIV)

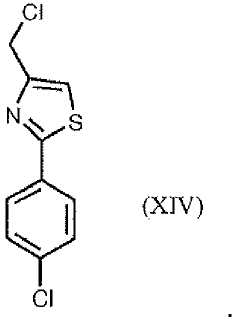

12. Procédé selon la revendication 10, **caractérisé en ce qu'**à l'étape 1, le produit intermédiaire de formule (III) est isolé, c'est-à-dire obtenu sous forme solide, avant sa mise en réaction ultérieure.

13. Procédé selon la revendication 10, **caractérisé en ce qu'**à l'étape 2, le Boc-alaninate protégé de formule (V) est isolé, c'est-à-dire obtenu sous forme solide, avant sa mise en réaction ultérieure.

14. Procédé selon la revendication 10, **caractérisé en ce qu'**à l'étape 3, le Boc-dialaninate protégé de formule (VII) obtenu n'est pas isolé, mais plutôt directement soumis à sa mise en réaction ultérieure.

15. Procédé selon la revendication 10, **caractérisé en ce qu'**à l'étape 1, le produit intermédiaire de formule (III) est isolé, c'est-à-dire obtenu sous forme solide, avant sa mise en réaction ultérieure.

16. Composé dichlorhydrate de L-alaninate de 2-{4-[2-({[2-(4-chlorophényl)-1,3-thiazol-4-yl]méthyl}sulfanyl)-3,5-dicyano-6-(pyrrolidin-1-yl)-pyridin-4-yl]phénoxy}éthyl-L-alanyle de formule (IX)

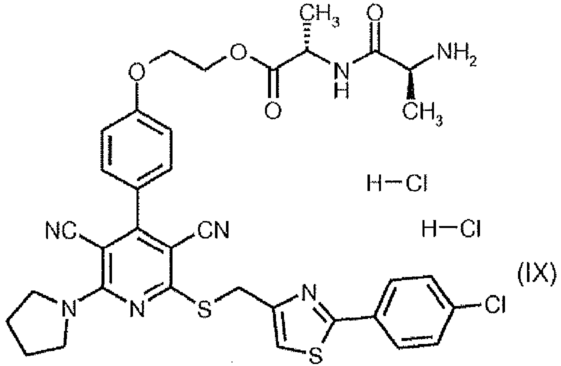

17. Composé dichlorhydrate de L-alaninate de 2-{4-[2-({[2-(4-chlorophényl)-1,3-thiazol-4-yl]méthyl}suifanyl)-3,5-dicyano-6-(pyrrolidin-1-yl)-pyridin-4-yl]phénoxy}éthyle de formule (VIII)
